# EUROPEAN PATENT APPLICATION

(11) **EP 4 467 080 A1**
(43) Date of publication of application: **27.11.2024**
(21) Application number: 23305807.2
(22) Date of filing: 22.05.2023
(51) Int. Cl.: A61B 6/12, A61B 6/00, A61B 8/08, A61B 8/12, A61B 8/00

(54) **FUSION OF EXTRALUMINAL AND INTRALUMINAL IMAGES USING ANATOMICAL FEATURE IN INTRALUMINAL IMAGE AND ASSOCIATED SYSTEMS, DEVICES, AND METHODS**

(71) Applicant: Koninklijke Philips N.V., 5656 AG Eindhoven (NL)
(72) Inventor: PAI RAIKAR, Vipul Shrihari, 5656 EINDHOVEN (NL); CATHIER, Pascal Yves François, 5656 EINDHOVEN (NL); TORJESEN, Alyssa, 5656 EINDHOVEN (NL); LAI, Marco, 5656 EINDHOVEN (NL); LEE, Brian C, 5656 EINDHOVEN (NL); MÜLLER, Manfred, 5656 EINDHOVEN (NL); UBACHS, René Leonardus Jacobus Marie, 5656 EINDHOVEN (NL)
(74) Representative: Philips Intellectual Property & Standards

(57) **Abstract**

A system includes a processor configured to receive extraluminal imaging data depicting an intraluminal imaging device positioned within a patient body. Based on the imaging data, the processor determines two candidate rotational orientations of the intraluminal imaging device. The processor also receives imaging data from the intraluminal device, and an extraluminal imaging device parameter. Based on this information, the processor identifies an anatomical feature depicted in the intraluminal imaging data and selects, based on the anatomical feature, one of the two candidate rotational orientations as a true rotational orientation of the imaging assembly. The processor then co-registers the intraluminal imaging data and the extraluminal imaging data based on the true rotational orientation, and displays the co-registered intraluminal imaging data and extraluminal imaging data.

## Description

### TECHNICAL FIELD

The present disclosure relates generally to ultrasound imaging devices and systems. In particular, the present disclosure provides systems and methods for determining the orientation of the ultrasound transducer so that ultrasound images can be fused with and/or registered onto X-ray images.

### BACKGROUND

Cardiac interventions done via transcatheter approaches have the potential to benefit a much wider cohort of patients who are typically ineligible for traditional surgical treatment options. These minimally invasive procedures have shown to be of similar efficacy as compared to surgery, they allow for shorter hospital stays and faster recovery, and have the potential to reduce the healthcare economic burden over time. This has led to a boom in development of various transcatheter devices designed to perform increasingly complex cardiac procedures, particularly for structural heart disease. These procedures are currently carried out by interventionalists who have a great deal of experience and are often limited to elite care centers.

A key enabler for these procedures is the availability of high-quality imaging and image guidance. Transcatheter approaches involve manipulation of complex devices under image guidance, primarily using X-ray fluoroscopy. While X-ray is great at visualizing devices and providing spatial context, it may not clearly visualize soft tissues. Consequently, a combination of X-ray and ultrasound imaging is key to visualizing critical tissue-device interactions. In addition, a key element of such interventions is the interpretation of a 3D workspace on 2D screens. Often, this contextual information is processed by the clinicians using their experience in manipulating the device and their deep understanding of anatomy.

Diagnostic and therapeutic ultrasound catheters have been designed for use inside many areas of the human body. For example, a trans-esophageal echocardiography (TEE) probe can be inserted into an esophagus of a patient and image the patient's heart with high frequency ultrasound waves. Another common diagnostic ultrasound method is intra-cardiac echocardiography (ICE), where a single rotating transducer or an array of transducer elements (sometimes referred to as a transducer array) is threaded through a patient's venous or arterial system (e.g., femoral vein access, jugular vein access, femoral artery access, etc.) and enters his or her heart to take images. Transducers on TEE probes and ICE catheters transmit ultrasound waves and receive echoes from the tissue. A signal generated from the echoes is transferred to a console which allows for the processing, storing, display, or manipulation of the ultrasound-related data.

TEE probes and ICE catheters are usually used along with the radiographic imaging device (e.g., a fluoroscopy system), to guide and facilitate medical procedures, such as transseptal lumen punctures, septal wall repair, left atrial appendage closures, atrial fibrillation ablation, and valve repairs. Real-time image fusion in the catheter lab is therefore of great value, as it allows the user to bring two (or more) separate imaging modalities into one common coordinate frame of reference. In the case of complex catheter based cardiac interventions, this allows operators to visualize the interventional device in X-ray while also seeing the interactions of the device with soft tissue in ultrasound imaging. This is traditionally done by registering the trans esophageal ultrasound (TEE) probe position in X-ray image field of view. A user can thus clearly see the device being manipulated on the X-ray but can also visualize the device interacting with soft tissue.

Typically, most interventional cardiac procedures utilize trans-esophageal echo (TEE), where a probe is inserted into an intubated patient under general anesthesia. However, with more recent developments in ultrasound transducer technology, it is possible to employ small-form-factor intraluminal probes. In order to enable image-fusion between x-ray and ultrasound, knowing the pose of the ultrasound transducer when visible in x-ray is vital. The challenge is that estimating the pose of these small-form-factor probes as opposed to larger form factor such as TEE, is more challenging when using X-ray imaging data alone. However, without reliable information of the orientation, fusion/co-registration of the two image streams may lose fidelity or fail entirely.

In order to co-register the ultrasound images from a TEE probe or an ICE catheter and radiographic images, a computer system has to identify the position and orientation of the ultrasound transducer. Knowing the 6 degree of freedom (6-DoF) pose of the ultrasound probe, the system can effectively overlay the generated ultrasound image onto the corresponding X-ray image. In case of TEE, because the diameter of a TEE probe can be as large as 13mm, the TEE probe and its internal structure can be discerned by the computer system in the radiographic images. However, in case of a mini TEE probe or an ICE catheter that can have a diameter of as little as 3 mm, while the computer system can determine its position, its rotational orientation may not be detectable with adequate precision in the radiographic images.

Thus, a need exists for systems and methods to determine the rotational (e.g., roll) orientation of intraluminal probes such as ICE catheters.

### SUMMARY

Disclosed is a position and orientation (e.g., pose) estimation system for intraluminal probes. The pose estimation system disclosed herein has particular, but not exclusive, utility for the co-registration and/or fusion of X-ray images captured from outside the body and intraluminal ultrasound images captured from inside the body. This disclosure describes systems and methods for resolving pose ambiguities for an intraluminal probe (e.g., an intracardiac echocardiography or ICE catheter or mini-transesophageal echocardiography or TEE probe), by utilizing information that is available inside the ultrasound imaging field of view when it is imaging known structures in a human heart or other organ. This system can receive, store, and process real-time data in the form of: 2D X-ray imaging, 2D and/or 3D ultrasound imaging, and the X-ray system's C-arm geometry and pose data. The system then processes X-ray images received from the C-arm to extract features of the ICE catheter's imaging assembly. However, because two possible solutions exist for the roll orientation of the probe based on X-ray imaging alone, the disclosed system interprets features of the ultrasound images as well to determine a complete 6-degree-of-freedom (6-DoF) pose. In particular, the anatomy depicted in the ultrasound images is used to determine which orientation the ICE catheter's imaging assembly is facing. For example, when anatomy that is expected to be depicted at a given orientation is actually depicted in ultrasound image, then system can determine that the ICE catheter's imaging assembly is facing that given orientation.

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. One general aspect includes a system that includes a processor circuit configured to: receive extraluminal imaging data obtained by an extraluminal imaging device, where the extraluminal imaging data depicts an intraluminal imaging device positioned within a patient body; determine, based on the extraluminal imaging data, a first rotational orientation and a second rotational orientation of an intraluminal imaging assembly around a longitudinal axis of the intraluminal imaging device, where the intraluminal imaging device may include the intraluminal imaging assembly; receive intraluminal imaging data obtained by the intraluminal imaging device; receive an extraluminal imaging device parameter associated the extraluminal imaging device obtaining the extraluminal imaging data; identify, based on the intraluminal imaging data and the extraluminal imaging device parameter, an anatomical feature depicted in the intraluminal imaging data; select, based on the anatomical feature, one of the first rotational orientation or the second rotational orientation for the imaging assembly around the longitudinal axis; perform image fusion of the intraluminal imaging data and the extraluminal imaging data based on the selected rotational orientation of the imaging assembly around the longitudinal axis; and output, to a display in communication with the processor circuit, the co-registered intraluminal imaging data and extraluminal imaging data. Other embodiments of this aspect include corresponding computer systems, apparatus, and computer programs recorded on one or more computer storage devices, each configured to perform the actions of the methods.

Implementations may include one or more of the following features. In some aspects, to select one of the first rotational orientation or the second rotational orientation, the processor circuit is configured to: identify an anatomical feature depicted in the intraluminal imaging data. In some aspects, the processor circuit is configured to select one of the first rotational orientation or the second rotational orientation based on motion of the anatomical feature in the intraluminal imaging data during rotation of the intraluminal imaging assembly around the longitudinal axis. In some aspects, the processor circuit is configured to use a first machine learning model to select one of the first rotational orientation or the second rotational orientation. In some aspects, the intraluminal imaging device may include intra-cardiac echocardiography (ICE) catheter, and where the intraluminal imaging assembly may include an ultrasound transducer array. In some aspects, the extraluminal imaging device may include a radiographic imaging device. In some aspects, the radiographic imaging device may include an x-ray imaging device. In some aspects, the extraluminal imaging device parameter may include at least one of: a pose of the extraluminal imaging device; or a geometry of the extraluminal imaging device. In some aspects, the extraluminal imaging device parameter may include an angle of the extraluminal imaging device along a cranial-caudal axis or a right-left anterior oblique axis. In some aspects, the processor circuit is configured to: determine, based on the radiographic imaging data: a position of the intraluminal imaging assembly in the radiographic imaging data; a rotational orientation of the intraluminal imaging assembly around a transverse axis of the intraluminal imaging device; and a rotational orientation of the intraluminal imaging assembly around a vertical axis of the intraluminal imaging device; and co-register the intraluminal imaging data and the extraluminal imaging data based on the position of the intraluminal imaging assembly, the rotational orientation of the intraluminal imaging assembly around the transverse axis, and the rotational orientation of the intraluminal imaging assembly around the vertical axis. In some aspects, the processor circuit is configured to use a second machine learning model to determine the position of the intraluminal imaging assembly, the rotational orientation of the intraluminal imaging assembly around the transverse axis, and the rotational orientation of the intraluminal imaging assembly around the vertical axis. In some aspects, to co-register the intraluminal imaging data and the extraluminal imaging data, the processor circuit is configured to overlay the intraluminal imaging data on the extraluminal imaging data, where, to output the co-registered intraluminal imaging data and extraluminal imaging data, the processor circuit is configured to output the intraluminal imaging data overlaid the extraluminal imaging data. In some aspects, the system may include the intraluminal imaging device. In some aspects, the processor circuit is configured for communication with the intraluminal imaging device, and where the processor circuit is configured to control the intraluminal imaging assembly to obtain the intraluminal imaging data. In some aspects, the system may include the extraluminal imaging device. In some aspects, the processor circuit is configured for communication with the extraluminal imaging device, and where the processor circuit is configured to control the extraluminal imaging device to obtain the extraluminal imaging data. Implementations of the described techniques may include hardware, a method or process, or computer software on a computer-accessible medium.

One general aspect includes a system that includes an intracardiac echocardiography (ICE) catheter configured to be positioned within a heart of a patient, where the ICE catheter may include an ultrasound transducer array configured to obtain ultrasound imaging data of the heart while the ice catheter is positioned within the heart; a radiographic imaging device configured to obtain radiographic imaging data of the heart, where the radiographic imaging device may include at least one of a pose or a geometry associated with obtaining the radiographic imaging data; and a processor circuit configured for communication with the ultrasound transducer array and the radiographic imaging device. The processor circuit is configured to: receive the radiographic imaging data from the radiographic imaging device, where the radiographic imaging data depicts the ice catheter while the ICE catheter is positioned within the heart; determine, based on the radiographic imaging data, a first candidate rotational orientation and a second candidate rotational orientation of the ultrasound transducer array around a longitudinal axis of the ice catheter as a result of a size of the ice catheter preventing determination of an actual/true rotational orientation of the ultrasound transducer array around the longitudinal axis based only the radiographic imaging data; receive the ultrasound imaging data from the ultrasound transducer array; receive at least one of the pose or the geometry of the radiographic imaging device; identify, based on the ultrasound imaging data and at least one of the pose or the geometry, an anatomical feature depicted in the ultrasound imaging data; select, based on the anatomical feature or motion of the anatomical feature, one of the first candidate rotational orientation or the second candidate rotational orientation as the actual/true rotational orientation of the ultrasound transducer array around the longitudinal axis; co-register the ultrasound imaging data and the radiographic imaging data based on the actual/true rotational orientation of the ultrasound transducer array around the longitudinal axis; and output, to a display in communication with the processor circuit, the co-registered ultrasound imaging data and radiographic imaging data. Other embodiments of this aspect include corresponding computer systems, apparatus, and computer programs recorded on one or more computer storage devices, each configured to perform the actions of the methods.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the pose determination system, as defined in the claims, is provided in the following written description of various embodiments of the disclosure and illustrated in the accompanying drawings.

### BRIEF DESCRIPTION OF THE DRAWINGS

Illustrative embodiments of the present disclosure will be described with reference to the accompanying drawings, of which:
**Fig. 1** is radiographic image showing an intraluminal ultrasound imaging probe and a catheter within a patient's body to image a region of interest, according to aspects of the present disclosure.
**Fig. 2** is a pictorial comparison of three different types of intraluminal ultrasound imaging probes, according to aspects of the present disclosure.
**Fig. 3** is a schematic, diagrammatic representation of a medical imaging system, according to aspects of the present disclosure.
**Fig. 4** is a is a diagrammatic view of an X-ray imaging device, according to aspects of the present disclosure.
**Fig. 5** is a diagrammatic side view of an intraluminal imaging device according to aspects of the present disclosure.
**Fig. 6** is a is a flow diagram of a method for displaying ultrasound imaging data and radiographic imaging data with respect to the region of interest within a body of a patient, according to aspects of the present disclosure.
**Fig. 7** is a block diagram showing at least a portion of an example pose estimation system, according to aspects of the present disclosure.
**Fig. 8** is a perspective view of the imaging assembly of the ultrasound imaging device in two substantially opposing orientations, according to aspects of the present disclosure.
**Fig. 9** is a schematic, diagrammatic illustration of the ultrasound imaging device with imaging assembly positioned on distal portion inserted into a heart of a patient in a first orientation, and proximal portion coupled to handle, according to embodiments of the present disclosure.
**Fig. 10** is a schematic, diagrammatic illustration of the imaging assembly being inserted into the heart in a second orientation different from the first orientation, according to aspects of the present disclosure.
**Fig. 11A** is a schematic, diagrammatic overview, in block diagram form, of a training mode for the object detector, according to aspects of the present disclosure.
**Fig. 11B** is a schematic, diagrammatic overview, in block diagram form, of a validation mode for the object detector, according to aspects of the present disclosure.
**Fig. 11C** is a schematic, diagrammatic overview, in block diagram form, of an inference mode or clinical usage mode for the object detector, according to aspects of the present disclosure.
**Fig. 12** is a diagram showing different views of the heart available as a function of C-arm geometry, according to aspects of the present disclosure.
**Fig. 13A** is a schematic, diagrammatic overview, in block diagram form, of a training mode for the ultrasound feature detector, according to aspects of the present disclosure.
**Fig. 13B** is a schematic, diagrammatic overview, in block diagram form, of a validation mode for the ultrasound feature detector, according to aspects of the present disclosure.
**Fig. 13C** is a schematic, diagrammatic overview, in block diagram form, of an inference mode or clinical usage mode for the ultrasound feature detector, according to aspects of the present disclosure.
**Fig. 14** is a schematic, diagrammatic view of the displacement of an image feature in the ultrasound imaging field of view of an intraluminal ultrasound imaging assembly during roll rotational motion, according to aspects of the present disclosure.
**Fig. 15** is a schematic, diagrammatic view of a roll angle disambiguation, according to aspects of the present disclosure.
**Fig. 16** is an X-ray image of an ongoing intracardiac interventional procedure, taken from outside the body, according to aspects of the present disclosure.
**Fig. 17** is screen display including a fused, co-registered X-ray and intraluminal image, of an ongoing intracardiac interventional procedure, according to aspects of the present disclosure.
**Fig. 18** is a schematic diagram of a processor circuit, according to embodiments of the present disclosure.

### DETAILED DESCRIPTION

This disclosure describes systems and methods for resolving pose ambiguities for an intraluminal probe (e.g., an ICE catheter or mini-TEE probe), by utilizing information that is available inside the ultrasound imaging field of view when it is imaging known structures in a human heart or other organ.

Central to accurate image fusion is the precise recovery of the pose of the ultrasound imaging probe in X-ray images. A challenge for doing this in small-form-factor probes is the ambiguity in recovering the rotation of the probe head about its long axis (e.g., the roll angle of the probe). Without accurate recovery of all six degrees of freedom, it is difficult to effectively overlay ultrasound images (whether 2D or 3D) onto the X-ray image stream with the correct position and orientation. Prior methods that provide pose optimization, enabling image fusion, were focused on large-form-factor probes where the geometry of the probe is much clearer in X-ray imaging, and where certain assumptions can be made about the orientation of the probe. For example, if a TEE probe located in the esophagus is imaging structures in the heart, then by definition the probe must be face-up with respect to an X-ray sensor located above the patient's chest.

To address this issue for intraluminal imaging catheters such as ICE probes, as well as smaller, more rotationally symmetrical TEE probes, the present disclosure provides algorithms to aid in the disambiguation of the pose of the ICE catheter from image-interpretation-based methods and C-arm parameters such as pose/geometry (also referred to as radiographic imaging device parameter) to obtain the 6 DoF pose of the ultrasound imaging element. The systems disclosed herein utilize the x-ray images generated of the intra-cardiac probe in addition to the pose of the C-arm to estimate a pair of possible poses of the catheter and an estimate of the anatomical view of the heart. Furthermore, this system learns to identify features of interest in the ultrasound image generated by the ICE catheter and track the motion of these features relative to any motion of the probe head, to resolve the pose ambiguity and track the rotation of the probe head.

In one aspect, the pose estimation system is a combination of software and hardware controllers. This system can receive, store, and process real-time data in the form of: 2D X-ray fluoroscopy imaging, 2D and/or 3D ultrasound imaging, and the X-ray system's C-arm geometry and pose data. In such aspects, the first part of the pipeline processes X-ray images received from the C-arm to extract salient features that represent the ICE probe head. This can be done using conventional image processing techniques. Possible approaches utilize pattern matching (detection) followed by pose estimation utilizing digitally reconstructed radiographs (DRR) from a high-resolution model of the transducer head. The pose is retrieved based on the best fitting DRR image and its associated transformation matrix. However, because the X-ray system sees "through" the ultrasound imaging probe, the same features are visible regardless of whether the probe is face-up toward the X-ray imager or face-down away from it. Thus, two possible solutions exist for the roll orientation of the probe - an ambiguity that cannot be resolved by the X-ray imaging alone.

An alternate approach is to utilize recent advances in modern data driven machine learning (ML) approaches. These methods, coupled with modern computational hardware, have enabled development of sophisticated pattern recognition algorithms. The object detection step described above can thus be achieved by utilizing an ML model (e.g., a convolutional neural network (CNN)) trained to detect the ICE transducer head based on clinical and synthetic data in a supervised fashion. An example of such a popular algorithm is YOLO. In the case of an ICE transducer, the probe head is of a small form factor and could benefit from up-sampling of the image resolution.

This up-sampling can be achieved by utilizing a CNN trained to extract image features and then learn network parameters to increase the resolution of the image and thus enhance features of the probe head. Following this optional step, the higher-resolution image is passed to a regression network which is trained and optimized to regress the pose of the probe head based on paired training with digitally reconstructed radiography (DRR) images of known transformations. The advantage of a data driven approach, taken by modern machine learning algorithms, is the ability to train on a vast cohort of data that can cover many corner cases, whereas in traditional approaches, the algorithms need to be tuned specifically to address these corner cases.

Once the 6-DoF pose of the transducer head is estimated, there is still a potential pose ambiguity, as described above. For example, as the transducer head is symmetric, the normal (surrogate for roll angle which is orthogonal along the long axis of the transducer) from the transducer face can have two solutions that are 180 degrees apart. To resolve this ambiguity, features as seen in the ultrasound image can be analyzed. As the anatomy of interest (e.g., the anatomy to be imaged by the ultrasound system) is known prior to the procedure, a feature detection and classification network can be utilized to identify anatomical features in 2D and/or 3D ultrasound images. In addition, the system may benefit from optimal C-arm angles known to image clinically relevant anatomical windows to the heart during interventional procedures. In an example, X-ray imaging within about 10 degrees of the optimal viewing angle provides good imaging results, as described below. This allows for the proper localization of anatomy in question in fluoroscopy images when the ICE catheter is positioned within the cardiac chambers. This can help in narrowing the search criteria for ultrasound feature detection. In addition, the features detected can also be tracked in the image (using methods such as optical flow) to recover the motion and direction of the rotation around the long axis of the transducer (e.g., the roll angle and roll rate). This allows the algorithm to select/identify the correct candidate/possibility, out of the two possible roll angle candidates for the normal of the transducer face, thus resolving the ambiguity in the 6-DoF pose.

The present disclosure aids substantially in the performance of minimally invasive interventional procedures, by improving real-time medical imaging through registration and image fusion of the X-ray and ultrasound image streams for small intraluminal (e.g., intracardiac) imaging probes. Implemented on a processor in communication with both an X-ray imaging system and an ultrasound probe, the pose estimation system disclosed herein provides the practical ability to understand the pose of the intraluminal (e.g., intracardiac) imaging probe, and therefore to fuse the two image streams. This augmented, fused image stream transforms a process requiring the surgeon's attention to two different image streams into one that provides a single, fused image stream, without the normally routine need to train clinicians to divide their attention. This unconventional approach improves the functioning of the medical imaging system, by permitting the fusion/registration of the two image streams in real time.

The pose estimation system may be implemented as a process at least partially viewable on a display, and operated by a control process executing on a processor that accepts user inputs from a keyboard, mouse, or touchscreen interface, and that is in communication with one or more imaging systems. In that regard, the control process performs certain specific operations in response to different inputs or selections made at different times. Certain structures, functions, and operations of the processor, display, sensors, and user input systems are known in the art, while others are recited herein to enable novel features or aspects of the present disclosure with particularity.

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. For example, while the pose estimation system is described in terms of ultrasound intracardiac imaging, it is understood that it is not intended to be limited to this application, but may be used in other circumstances where the fusion/registration of two different image streams relies on knowledge of the 6-DoF pose of an imaging probe. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

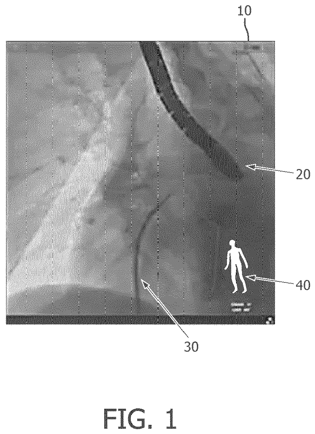

**Fig. 1** is radiographic image 10 showing an intraluminal ultrasound imaging probe 20 and a catheter 30 within a patient's body to image a region of interest, according to aspects of the present disclosure. In this case, the region of interest is within the patient's heart. In some instances, the radiographic image 10 is captured by a radiography imaging device, such as a fluoroscopy system and is displayed on a display device. In some aspects, the intraluminal ultrasound imaging probe 20 may for example be a transesophageal echocardiogram (TEE) probe inserted into the patient's esophagus through the patient's mouth. Sometimes, if the circumstances require, the TEE probe 20 can be inserted all the way into the patient's stomach to image the patient's heart at a preferable angle. A catheter 30, such as an intracardiac echography (ICE) probe, enters the patient's body through his or her femoral or jugular vein and is threaded to the patient's heart. When a catheter such as catheter 30 enters the patient's body through his/her jugular vein, the catheter 30 is advanced downward into the patient's heart through the superior vena cava. However, when catheter 30 enters the patient's body through his/her femoral vein, the catheter would be advanced upward into the patient's heart through the inferior vena cava.

As illustrated in Fig. 1, the radiographic imaging device that captures the radiographic image 10 is directed at the region of interest from outside of the patient's body while the TEE probe 20 and the catheter 30 is positioned adjacent to or within the patient's heart. Due to the difference in dimensions of the esophagus and vein that allow passage of a regular TEE probe, such as the TEE probe 20, and catheter 30, the TEE probe 20 has a diameter substantially larger than that of the catheter 30. External characteristics of the TEE probe 20 can be readily identified by a computer system that processes radiographic images from the radiographic imaging device. In some implementations, X-ray from the radiographic imaging device penetrates certain outer radiolucent features of the TEE probe 20 and allows the computer system to discern inner radiopaque structures of the TEE probe 20. Because the computer system can identify features and characteristics of the TEE probe 20, the computer system can determine a position and an orientation of the TEE probe 20. The same cannot be said for the catheter 30. Given the resolution of the radiographic imaging data, while the position of catheter 30 is readily identifiable, the smaller dimensions of catheter 30 may not provide enough discernible features and characteristics for the computer system to determine the orientation of catheter 30. In some instances, the orientation refers to the rotational orientation around a longitudinal direction along the length of catheter 30. For example, the silhouette of the catheter 30 in the radiographic image 10 provides little detail. In contrast, the silhouette of the TEE probe 20 reveals much more detail, including the gaps between vertebrae embedded within a polymer jacket. In some examples, a figurine icon 40 is also displayed to indicate the imaging plane of the displayed radiographic image relative to the patient's body.

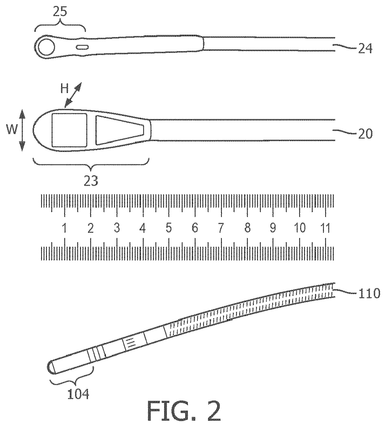

**Fig. 2** is a pictorial comparison of three different types of intraluminal ultrasound imaging probes, according to aspects of the present disclosure. In the example shown in Fig. 2, the distal portion 23 of a trans-esophageal imaging (TEE) probe 20 has a width W that is greater than its height H, thus providing features that can distinguish the roll orientation of the TEE probe 20 in an X-ray image. Disambiguation of two different roll angle solutions can be performed by assumption; if the TEE probe is located in the esophagus and is imaging the heart, then it must be face-up with respect to an X-ray detector located above the heart.

However, the distal portion 25 of a mini-TEE probe 24 is both smaller and more rotationally symmetric than the distal portion 23 of the TEE probe 22, thus making it harder to determine the roll angle of the mini-TEE 24 probe based on X-ray imaging alone. Similarly, the distal portion 104 of an ICE catheter 110 may be even smaller (e.g., a diameter between 9.4 mm and 3 mm, or smaller), and may be substantially rotationally symmetrical, thus making it extremely difficult to determine the roll angle of the ICE catheter 110 from X-ray imaging.

The present application is therefore directed to determination of the roll angle of intraluminal imaging devices, such as mini-TEE probes and intracardiac echography (ICE) catheters, from a combination of X-ray and ultrasound imaging data.

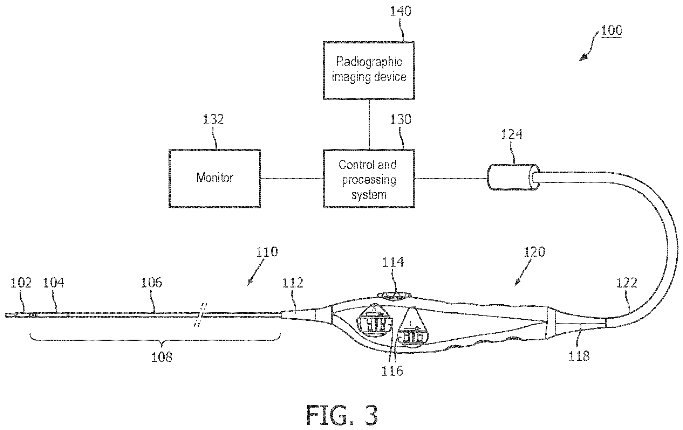

**Fig. 3** is a schematic, diagrammatic representation of a medical imaging system 100, according to aspects of the present disclosure. The system 100 can include an ultrasound imaging device 110, a connector 124, a control and processing system 130 (for example, a console and a computer), and a monitor 132. In some embodiments, the ultrasound imaging device 110 is an ICE catheter. The ultrasound imaging device 110 includes an imaging assembly 102 at the tip of a flexible elongate member 108, and a handle 120. The flexible elongate member 108 includes a distal portion 104 and a proximal portion 106. The distal end of the distal portion 104 is attached to the imaging assembly 102. The proximal end of the proximal portion 106 is attached to the handle 120, for example, by a resilient strain reliever 112. The handle 120 may be used for manipulation of the ultrasound imaging device 110 and manual control of the ultrasound imaging device 110. The imaging assembly 102 can include an imaging core with ultrasound transducer elements and associated circuitry. The handle 120 can include actuators 116, a clutch 114, and other steering control components for steering the ultrasound imaging device 110. The steering may include deflecting the imaging assembly 102 and the distal portion 104 along different planes, as described in greater details herein.

The handle 120 is connected to the connector 124 via another strain reliever 118 and a connection cable 122. The connector 124 may be configured to provide suitable configurations for interconnecting the control and processing system 130 and the monitor 132 to the imaging assembly 102. The control and processing system 130 may be used for processing, storing, analyzing, and manipulating data, and the monitor 132 may be used for displaying obtained signals generated by the imaging assembly 102. The control and processing system 130 can include one or more processors, memory, one or more input devices, such as keyboards and any suitable command control interface device. The control and processing system 130 can be operable to facilitate the features of the medical imaging system 100 described herein. For example, a processor can execute computer readable instructions stored on the non-transitory tangible computer readable medium. The monitor 132 can be any suitable display device, such as a liquid-crystal display (LCD) panel or the like.

Each of the actuators 116 is attached to one or more distally-extending pull wires that are threaded through the flexible elongate member 108 and anchored to the distal portion 104.

In operation, a physician or a clinician may advance the flexible elongate member 108 through a blood vessel into a patient's heart (e.g., a chamber of the patient's heart). By controlling the actuators 116 and the clutch 114 on the handle 120, the physician or clinician can steer the flexible elongate member 108 to a position near the region of interest to be imaged. For example, one actuator 116 may deflect the imaging assembly 102 and the distal portion 104 in along a first plane and the other actuator 116 may deflect the imaging assembly 102 and the distal portion 104 along a second plane not parallel to the first plane. In some embodiments, the first plane has a normal direction perpendicular to the normal direction of the second plane. The clutch 114 provides a locking mechanism to lock the positions of the actuators 116 and in effect lock the deflection of the flexible elongate member while imaging the region of interest. Aspects of the present disclosure can include steering mechanism features similar to those described in U.S. Provisional App. No. 62/402,483, filed September 30, 2016, the entirety of which is hereby incorporated by reference herein. Additionally, embodiments of the present disclosure can include features similar to those described in U.S. Provisional App. No. 62/403,479, filed October 3, 2016, U.S. Provisional App. No. 62/434,517, filed December 15, 2016, U.S. Provisional App. No. 62/403,311, filed October 3, 2016, U.S. Provisional App. No. 62/437,778, filed December 22, 2016, U.S. Provisional App. No. 62/401,464, filed October 29, 2016, U.S. Provisional App. No. 62/401,686, filed October 29, 2016, and/or U.S. Provisional App. No. 62/401,525, filed October 29, 2017, the entireties of which are hereby incorporated by reference herein.

The imaging process may include activating the ultrasound transducer elements on the imaging assembly 102 to produce ultrasonic energy. A portion of the ultrasonic energy is reflected or scattered by the region of interest and the surrounding anatomy, and the ultrasound echo signals are received by the ultrasound transducer elements. The connector 124 transfers the received echo signals in the form of ultrasound imaging data to the control and processing system 130 where the ultrasound image is reconstructed and displayed on the monitor 132. In some embodiments, the control and processing system 130 can control the activation of the ultrasound transducer elements and the reception of the echo signals. In some embodiments, the control and processing system 130 and the monitor 132 may be part of a same system. In some instances, the ultrasound image can be further displayed in a different monitor (not shown in Fig. 2) to be viewed by a physician or clinician.

The system 100 may be utilized in a variety of applications such as trans-septal punctures, left atrial appendage closures, atrial fibrillation ablation, and valve repairs and can be used to image vessels and structures within a living body. In some examples, the device 110 can be sized and shaped, structurally arranged, and/or otherwise configured to be positioned within any suitable anatomy and/or body lumen of a patient. For example, the device 110 can be an intraluminal device. Although the system 100 is described in the context of intraluminal imaging procedures, the system 100 is suitable for use with any catheterization procedure, e.g., ICE, mini TEE. In addition, the imaging assembly 102 may include any suitable imaging sensor, including but not limited to 2D ultrasound, 3D ultrasound, optical coherence tomography (OCT) or other electro-optical sensor, or visible light camera.

In some embodiment, the ultrasound imaging device 110 includes a flexible elongate member 108 that can be positioned within a vessel, such as a femoral vein or a jugular vein. The flexible elongate member 108 may have a distal portion 104 and a proximal portion 106. The ultrasound imaging device 110 includes an imaging assembly 102 that is mounted within the distal portion 104 of the flexible elongate member 108.

In some embodiments, the medical imaging system 100 is used for generating 2D and 3D images. In some examples, the medical imaging system 100 is used for generating multi-plane images in two or more different planes at some non-zero angle or distance to each other.

In some embodiments, the control and processing system 130 is in communication with an extraluminal imaging device 140. In some embodiments, the extraluminal imaging device 140 is a radiographic imaging device (e.g., an x-ray imaging device), such as a fluoroscopy or angiography device. The control and processing system 130 can be used to activate, operate, and control the radiographic imaging device 140. In some embodiments, the control and processing system 130 may be used for processing, storing, analyzing, and manipulating radiographic imaging data received from the radiographic imaging device 140, and the monitor 132 may be used for displaying obtained radiographic imaging data generated by the radiographic imaging device 140. Radiographic imaging data is one example of extraluminal imaging data. Radiographic imaging data can include x-ray imaging data, computed tomography (CT) imaging data, etc. Other examples of extraluminal imaging data can include magnetic resonance imaging (MRI), etc. The control and processing system 130 can include one or more processors, memory, one or more input devices, such as keyboards and any suitable command control interface device. The control and processing system 130 can be operable to facilitate the features of the medical imaging system 100 described herein. For example, a processor can execute computer readable instructions stored on the non-transitory tangible computer readable medium.

In some aspects, the control and processing system 130 outputs both ultrasound imaging data received from the ultrasound imaging device 110 and the radiographic imaging data received from the radiographic imaging device 140, to the monitor 132 for simultaneous display of the ultrasound imaging data and the radiographic imaging data. In some instances, the ultrasound imaging data and the radiographic imaging data are displayed on the monitor 132 in real time while the ultrasound imaging device 110 and the radiographic imaging device 140 are capturing them within the patient's body.

In some embodiments, the ultrasound imaging data and the radiographic imaging data are co-registered before they are displayed on the monitor. To co-register the ultrasound imaging data and the radiographic imaging data, the control and processing system 130 has to identify the position and orientation of the ultrasound imaging device 110 and the radiographic imaging device 140. The orientation, field of view, and position of the radiographic imaging device 140 is readily available to the control and processing system 130 as the movement of the radiographic imaging device 140 is controlled through the control and processing system 130. In some embodiments where fluoroscopy system is used, the radiographic imaging element orbits around the patient's body along a C-shaped arm (C-arm) and the orbiting is controlled through the control and processing system 130.

With respect to the position of the ultrasound imaging device 110, the control and processing system 130 can determine the position of the imaging assembly 102 based on radiographic imaging data obtained by the radiographic imaging device 140. To achieve that, the radiographic imaging device 140 is directed at the region of interest from outside of the patient's body while the imaging assembly 102 of the ultrasound imaging device 110 is advanced into and positioned within the region of interest. In some examples, the region of interest is the patient's heart. The foregoing arrangement allows the radiographic imaging device 140 to obtain radiographic imaging data that contain information of the position of the imaging assembly 102. For example, such information includes distances between the imaging assembly 102 and two reference points adjacent to the region of interest within the patient's body. Oftentimes the imaging assembly 102 is too small for the control and processing system 130 to reliably determine the orientation of the imaging assembly 102. Having obtained or received positions and orientations of the ultrasound imaging device 110 and the radiographic imaging device 140, the control and processing system 130 can co-register the ultrasound imaging data and the radiographic imaging data and output the co-registered imaging data to the monitor 132 for display. When displayed on the monitored 132, the co-registered imaging data are displayed such that the ultrasound image and the radiographic image share substantially the same field of view. In some embodiments, the control and processing system 130 can overlay the ultrasound image on top of the radiographic image to provide a more intuitive view for a physician or clinician performing the catheterization.

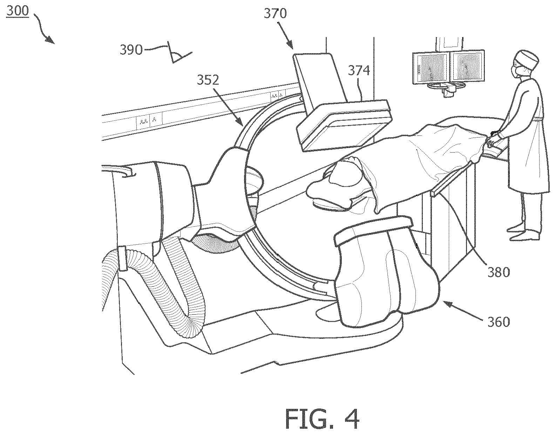

**Fig. 4** is a is a diagrammatic view of an X-ray imaging device, according to aspects of the present disclosure. The X-ray imaging device 300 may for example be the radiographic imaging device 140 of Fig. 3, or may be a different device. The X-ray imaging device 300 may be of any suitable type, for example, it may be a stationary X-ray system such as a fixed C-arm X-ray device, a straight arm X-ray device, or a u-arm device. The X-ray imaging device 300 may additionally be any suitable mobile device such as a mobile C-arm X-ray device. The X-ray imaging device 300 may also be in communication with the X-ray angiography control and processing system 130 of Fig. 3. In some aspects, the X-ray device 300 may include a digital radiography device or any other suitable device.

The X-ray imaging device 300 as shown in Fig. 4 includes an X-ray source 360, a detector 370 including an X-ray input screen 374. The X-ray source 360 and the input screen 374 may be mounted at a mutual distance and mounted on a movable arm 352 such as a C-arm. Positioned between the X-ray source 360 and the X-ray detector 370 may be an anatomy of a patient or object 380. The X-ray imaging device 300 may be used to image any suitable location or region of a patient's anatomy, including tissues, organs, malignancies, or any other structures or features. For example, the X-ray imaging device 300 may image without limitation the liver, heart, kidneys, gall bladder, pancreas, lungs; ducts; intestines; nervous system structures including the brain, dural sac, spinal cord and peripheral nerves; the urinary tract; as well as valves within the blood vessels, blood, chambers or other parts of the heart, abdominal organs, and/or other systems of the body. The imaging device 300 may additionally image tumors, cysts, lesions, hemorrhages, or blood pools, muscle, blood, blood plasma, interstitial fluid, lymph plasma, cerebrospinal fluid, intraocular fluid, serous fluid, synovial fluid, digestive fluid, urinary fluid, amniotic fluid, or any other type of suitable fluid, or any other region, structure, fluid, or gas within a patient anatomy.

The X-ray source 360 may include an X-ray tube adapted to generate X-rays. Some aspects of the X-ray source 360 may include one or more vacuum tubes including a cathode in connection with the negative lead of a high-voltage power source and an anode in connection with the positive lead of the same power source. The cathode of the X-ray source 360 may additionally include a filament. The filament may be of any suitable type or constructed of any suitable material, including tungsten or rhenium tungsten, and may be positioned within a recessed region of the cathode. One function of the cathode may be to expel electrons from the high voltage power source and focus them into a well-defined beam aimed at the anode. The anode may also be constructed of any suitable material and may be configured to create X-ray radiation from the emitted electrons of the cathode. In addition, the anode may dissipate heat created in the process of generating X-ray radiation. The anode may be shaped as a beveled disk and, in some embodiments, may be rotated via an electric motor. The cathode and anode of the X-ray source 360 may be housed in an airtight enclosure, sometimes referred to as an envelope.

In some embodiments, the X-ray source 360 may include a radiation object focus which influences the visibility of an image. The radiation object focus may be selected by a user of the system 100 or by a manufacturer of the system 100 based on characteristics such as blurring, visibility, heat-dissipating capacity, or other characteristics. In some embodiments, an operator or user of the system 100 may switch between different provided radiation object foci in a point-of-care setting.

The detector 370 may be configured to acquire X-ray images and may include the input screen 374. The input screen 374 may include one or more intensifying screens configured to absorb X-ray energy and convert the energy to light. The light may in turn expose a film or activate a light detector such as a charge coupled device (CCD) camera. The input screen 374 may be used to convert X-ray energy to light in embodiments in which the film may be more sensitive to light than x-radiation. Different types of intensifying screens within the image intensifier may be selected depending on the region of a patient to be imaged, requirements for image detail and/or patient exposure, or any other factors. Screens may be constructed of any suitable materials, including barium lead sulfate, barium strontium sulfate, barium fluorochloride, yttrium oxysulfide, or any other suitable material. The input screen 374 may be a fluorescent screen or a film positioned directly adjacent to a fluorescent screen. In some embodiments, the input screen 374 may also include a protective screen to shield circuitry or components within the detector 370 from the surrounding environment. The X-ray detector 370 may additionally be referred to as an X-ray sensor.

The object 380 may be any suitable object to be imaged. In an exemplary embodiment, the object 380 may be the anatomy of a patient including any region of a patient's anatomy previously mentioned. More specifically, the anatomy to be imaged may include the coronary region. In some embodiments, the object 380 may include man-made structures.

In some embodiments, the X-ray source 360 and the X-ray detector 370 are mounted to the movable arm 352. In this configuration, the X-ray source 360 and the X-ray detector 370 may be rotated around the object 380 or patient anatomy to acquire images of the object 380 or patient anatomy at different angles. The movable arm 352 may move the X-ray source 360 and detector 370 to any suitable location around the object 380 or patient anatomy. In some embodiments, the movable arm 352 may receive command signals from the system 154 or 164 based on a user input to move the X-ray source 360 and detector 370 to a desired position or angle 390 with respect to the object 380 to be imaged. The arm 352 may be of any suitable type or shape in addition to the one shown in Fig. 4 and may additionally be referred to as a gantry. In some embodiments, the X-ray imaging device 300 may include more than one set of X-ray sources 360 and detectors 370. For example, the X-ray imaging device 300 may be a bi-plane X-ray imaging system. In embodiments in which the X-ray imaging device 300 includes multiple sets of X-ray sources 360 and corresponding X-ray detectors 370, a physician may image the same regions of a patient's anatomy from multiple angles simultaneously or may image different regions of the patient's anatomy simultaneously.

As previously mentioned, the X-ray imaging device 300 may be configured to acquire x-ray image (e.g., fluoroscopy or angiography images). In such embodiments, a contrast agent may be introduced to a patient's anatomy before imaging. The contrast agent may be used to enhance the visibility of internal structures within a patient's anatomy. The contrast agent may absorb external X-rays, resulting in decreased exposure on the X-ray detector 370. In other embodiments, in which fluoroscopy images are to be obtained, a contrast agent may not be introduced to the patient anatomy prior to imaging. The contrast agent may be of any suitable type previously listed.

When an X-ray processing system, such as the control and processing system 130 of Fig. 3, is in communication with the X-ray imaging device 300, various data may be transmitted. This communication includes X-ray imaging data as well as control commands to the X-ray source 360 and/or X-ray detector 370 of the X-ray device 300. In some embodiments, the X-ray imaging device 300 may perform preliminary processing of the X-ray data prior to relaying the data to the processing system. In examples of such embodiments, the X-ray imaging device 300 may perform amplification, filtering, and/or aggregating of the data. In an embodiment, the X-ray image processing system may also supply high- and low-voltage direct current (DC) power to support operation of the device 300 including circuitry within the device.

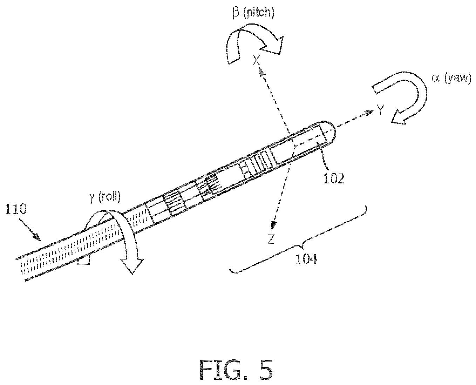

**Fig. 5** is a diagrammatic side view of an intraluminal imaging device 110 according to aspects of the present disclosure. The intraluminal imaging device has a 6-degree-of-freedom (6-DoF) pose than includes an X, Y, and Z position in the X-ray system's coordinate frame, as well as a rotational orientation around each of the X, Y, and Z axes. The Y-axis forms the longitudinal axis of the intraluminal imaging device 110, the X-axis forms a transverse axis of the intraluminal imaging device 110, and Z-axis forms a vertical axis of the intraluminal imaging device 110. The X-axis, Y-axis, and the Z-axis are perpendicular and/or orthogonal to one another. For purposes of this disclosure, rotational orientation around the Z-axis is referred to as a pitch angle b, while rotational orientation around the X-axis is referred to as a yaw angle a. Rotational orientation around the Y-axis or longitudinal axis is referred to as a roll angle g.

The distal portion 104 of the intraluminal imaging device 110 includes an imaging assembly 102, which can potentially be seen and identified in an X-ray image stream of sufficiently high resolution. However, as described above, since the X-ray imager sees "though" the imaging assembly 102, any determination of the roll angle g results in two candidate/possible solutions, a first solution g1 for a first possible orientation (e.g., face-up) and a second solution g2 for a second possible orientation (e.g., face-down) that is 180 degrees away from g1. In other words, an imaging assembly 102 at the first orientation g1 may be virtually indistinguishable in the X-ray image from an imaging assembly 102 at the second orientation g2. Disambiguation of the first solution g1 and second solution g2 into a single solution g may thus be extremely challenging using X-ray imaging data alone.

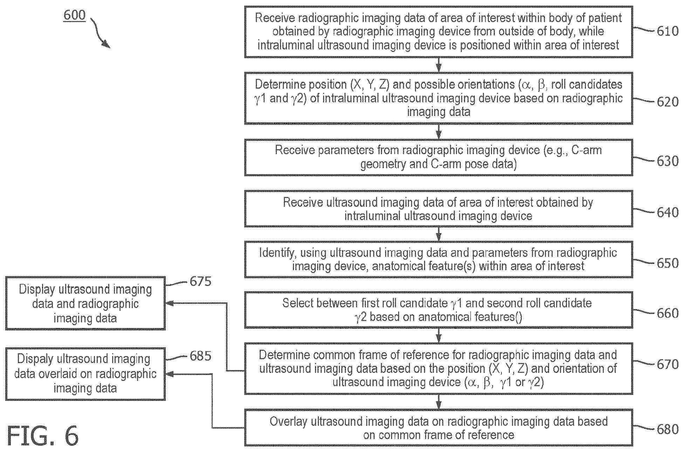

**Fig. 6** is a is a flow diagram of a method 600 for displaying ultrasound imaging data and radiographic imaging data with respect to the region of interest within a body of a patient, according to aspects of the present disclosure. It is understood that the steps of method 600 may be performed in a different order than shown in Fig. 6, additional steps can be provided before, during, and after the steps, and/or some of the steps described can be replaced or eliminated in other embodiments. One or more of steps of the method 600 can be carried by one or more devices and/or systems described herein, such as components of the medical imaging system 100 and/or processor circuit 1750.

In step 610, the method includes receiving extraluminal imaging data, such as radiographic imaging data (e.g., fluoroscopic X-ray data), of a region of interest within the body of a patient. The radiographic imaging data is obtained by a radiographic imaging device from outside of the body, while an intraluminal ultrasound imaging device is positioned within the region of interest, or adjacent to the region of interest such that the region of interest falls within the field of view of the intraluminal ultrasound imaging device. The extraluminal imaging data depicts an intraluminal imaging device positioned within a patient body and an intraluminal imaging assembly of the intraluminal imaging device in either a first rotational orientation or a second rotational orientation around a longitudinal axis of the intraluminal imaging device.

In step 620, the method includes determining the position (X, Y, Z), orientation (a, b), and possible candidate orientations (roll candidates γ1 and γ2) of the intraluminal ultrasound imaging device, based on the radiographic imaging data. The roll candidates γ1 and γ2 can be referred to as the first rotational orientation or the second rotational orientation around the longitudinal axis of the intraluminal imaging device. This determination may for example involve either classical image processing techniques, a machine learning (ML) classification, bounding box detection, or segmentation model such as a CNN, or combinations thereof. In some instances, this determination may be made using only the radiographic imaging data (e.g., not using the radiographic imaging device parameter and/or not using the intraluminal imaging data).

Examples of classification networks can be found for example in U.S. Provisional Patent Application No. 63/293,232, filed December 23, 2021, entitled "Methods and systems for clinical scoring a lung ultrasound" and U.S. Provisional Patent Application No. 63/294501, filed December 29, 2021, entitled "Machine-learning image processing independent of reconstruction filter", each of which is incorporated by reference as though fully set forth herein. An example of bounding box object detection can be found in Indian Patent Application No. 202141034243, filed July 29, 2021, entitled "Generating location data" (International Application No. PCT/EP2022/070410), which is incorporated by reference as though fully set forth herein. Examples of image or video segmentation can be found for example in U.S. Provisional Patent Application No. 63/325,660, filed March 31, 2022, entitled "Methods and systems for ultrasound-based structure localization using image and user inputs", and U.S. Publication No. 2022/0198669, filed March 30, 2020, entitled "Segmentation and view guidance in ultrasound imaging and associated devices, systems, and methods", each of which is incorporated by reference as though fully set forth herein. Moreover, the present disclosure can include aspects described with respect to deep-learning based ultrasound imaging, in U.S. Provisional App. No. 63/127,429, filed December 18, 2020, titled "ULTRASOUND IMAGE-BASED IDENTIFICATION OF ANATOMICAL SCAN WINDOW, PROBE ORIENTATION, AND/OR PATIENT POSITION" (Attorney Docket No. 2020PF00686 / 44755.2169PV01), which is incorporated by reference as though fully set forth herein.

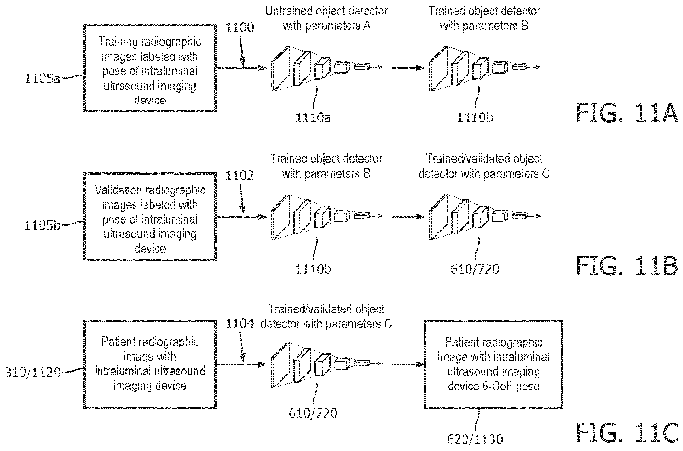

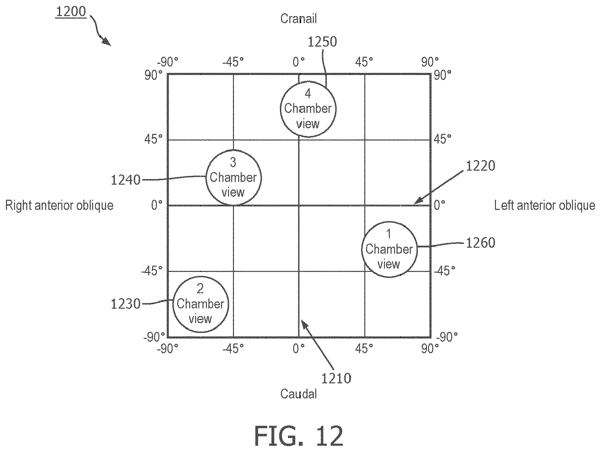

Machine learning is discussed below in more detail in Figs. 11A-11C. In step 630, the method includes receiving parameters from the radiographic imaging device, such as C-arm geometry and/or C-arm pose data, described below in greater detail in Fig. 12.

In step 640, the method includes receiving ultrasound imaging data of the region of interest. This imaging data is obtained by the intraluminal ultrasound imaging device.

In step 650, the method includes identifying, using the ultrasound imaging data and the parameters from radiographic imaging device, anatomical feature(s) within region of interest. This may for example be done with classical image processing or image recognition techniques, or may involve another ML model such as a convolutional neural network. For example, step 650 may involve identifying, in real time, specific anatomical features of the heart obtained by an ICE catheter.

However, the search criteria for any image recognition algorithm or ML classification/segmentation model can be narrowed considerably without knowledge of the C-arm geometry of the radiographic imaging system, as this provides information about soft tissue structures that exist within the field of view of the radiographic imaging system, as well as the 5-DoF pose (X, Y, Z, a, b) of the intraluminal ultrasound imaging device within the field of view of the radiographic imaging system. With this knowledge, it is possible to exclude anatomical features that would not be visible to the intraluminal ultrasound imaging device, and identify only those features that may be within the ultrasound imaging field of view. This may reduce the computational burden on the image recognition algorithm or ML classification/segmentation model, as well as improving the accuracy of the identification.

In step 660, the method includes selecting between the first roll candidate γ1 and the second roll candidate γ2. For example, one of the first rotational orientation or the second rotational orientation around the longitudinal axis (e.g., two possibilities depicted in the extraluminal/radiographic imaging data) is selected or identified for the intraluminal imaging assembly. The selected rotational orientation can be referred to as the actual or true rotational orientation around the longitudinal axis. In some aspects, this determination can be made only/purely based on the identified features in the current 2D or 3D ultrasound image of the real-time ultrasound image stream. For example, if the ultrasound probe is located in the right atrium and can see the mitral valve, then the ultrasound transducer array is presumed to be facing in an anterior direction with respect to the patient (e.g., upward if the patient is lying on their back). In other aspects, as described below, the roll orientation of the ultrasound imaging probe may be disambiguated by determining how features within the ultrasound image stream (whether identified or not) move within the image as the ultrasound probe is rotated around its longitudinal axis. In still other aspects, various combinations of feature identification, motion analysis, and X-ray imagery may be used. For example, analysis of the X-ray imagery may be used to obtain two candidate roll orientations g1 and g2 within a certain error tolerance, and then motion analysis may be used to disambiguate between the g1 and g2 solutions. Finally, the identification of specific features within the image, coupled with detailed knowledge of where the X-ray imaging system is pointed, can be used to reduce the error tolerance. Depending on the implementation, other combinations or sequences of these techniques may be used instead or in addition. In some instances, this determination may be made using only the radiographic imaging data (e.g., not using the radiographic imaging device parameter and/or not using the intraluminal imaging data).

The method includes performing image fusion and/or co-registration of the intraluminal imaging data and the extraluminal imaging data. For example, performing image fusion and/or co-registration is at least partially based on the selected/identified rotational orientation (can be referred to as the actual or true rotational orientation) of the imaging assembly around the longitudinal axis. Performing image fusion and/or co-registration is also based on the position (X, Y, Z) and the orientation of the ultrasound imaging device (α, β) around the transverse axis and the vertical axis.

Co-registering and/or fusing can include, in step 670, determining a common frame of reference for radiographic imaging data and ultrasound imaging data based on the position (X, Y, Z) and the orientation of the ultrasound imaging device (α, β, one of γ1 or γ2). Examples of image fusion and/or co-registration techniques may for example be found in U.S. Patent No. 10,238,361, titled "Combination of ultrasound and x-ray systems", U.S. Patent No. 10,157,491, titled "Real-time scene-modeling combining 3D ultrasound and 2D X-ray imagery", which are incorporated by reference as though fully set forth herein.

In step 675, the method includes displaying the ultrasound imaging data and the radiographic imaging data on a display. In an example, the ultrasound imaging data and the radiographic imaging data are each displayed in a different window on the display.

In step 680, the method includes overlaying the ultrasound imaging data on the radiographic imaging data based on the common frame of reference. In some instances, this step may be considered part of the image fusion/co-registration process described above.

In step 685, the method includes displaying the ultrasound imaging data overlaid on the radiographic imaging data. This may for example allow a detailed, colorized image stream of soft tissues to be overlaid on a black-and-white radiographic image stream that clearly shows the structure, location, and pose of the ultrasound imaging device, as well as any interventional devices located within the region of interest. This combined image may be of particular value in assessing the interaction of an interventional device with soft tissue, such as for example when a mitral valve clip is being positioned over one or more leaflets of a mitral valve.

It is noted that flow diagrams are provided herein for exemplary purposes; a person of ordinary skill in the art will recognize myriad variations that nonetheless fall within the scope of the present disclosure. For example, the logic of flow diagrams may be shown as sequential. However, similar logic could be parallel, massively parallel, object oriented, real-time, event-driven, cellular automaton, or otherwise, while accomplishing the same or similar functions. In order to perform the methods described herein, a processor may divide each of the steps described herein into a plurality of machine instructions, and may execute these instructions at the rate of several hundred, several thousand, several million, or several billion per second, in a single processor or across a plurality of processors. Such rapid execution may be necessary in order to execute the method in real time or near-real time as described herein. For example, the calculations or ML interactions required to determine the 6-DoF pose of the ultrasound probe, along with the identification of features in the ultrasound image stream and the fusion/co-registration of the radiographic and ultrasound image streams, may need to occur multiple times per second (e.g., at 30 Hz or 60 Hz) in order to provide real-time feedback to the clinician.

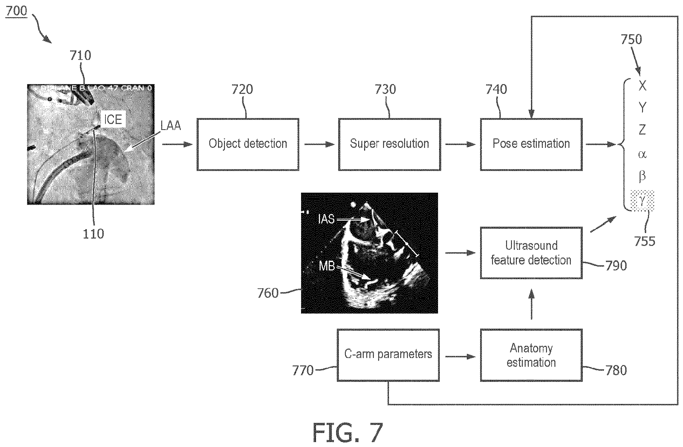

**Fig. 7** is a block diagram showing at least a portion of an example pose estimation system 700, according to aspects of the present disclosure. In the example shown in Fig. 7, a radiographic image 710 (e.g., a current frame from a real-time X-ray fluoroscopic image stream) is received by an object detection module 720, where the intraluminal ultrasound imaging device 110 is identified. The image is then optionally received by a super-resolution module 730 for up-sampling to a higher resolution, which may air in identifying specific features of the intraluminal ultrasound imaging device such as the imaging assembly or transducer array. Next, the up-sampled image is received by a pose estimation module 740, which identifies the 6-DoF pose 750 (X, Y, Z, a, b, g) of the intraluminal imaging device 110 as described above. At this stage, the g value 755 remains ambiguous, and is represented by two candidate values g1 and g2 as described above.

In one aspect, the pose estimation module 740 can receive, store, and process real-time data in the form of 2D X-ray fluoroscopy imaging and the X-ray system's C-arm geometry and pose data. In such aspects, the pose estimation module processes X-ray images received from the C-arm to extract salient features that represent the ICE probe head. This can be done using conventional image processing techniques. Possible approaches utilize pattern matching (detection) followed by pose estimation utilizing digitally reconstructed radiographs (DRR) from a high-resolution model of the transducer head. The pose is retrieved based on the best fitting DRR image and its associated transformation matrix. However, because the X-ray system sees "through" the ultrasound imaging probe, the same features are visible regardless of whether the probe is face-up toward the X-ray imager or face-down away from it. Thus, two possible solutions g1 and g2 exist for the roll orientation of the probe - an ambiguity that cannot be resolved by the X-ray imaging alone.

In a sequential or parallel stage, an ultrasound image 760 (e.g., the most current frame of a real-time image stream captured by the intraluminal imaging device 110) is received by an ultrasound feature detection module 790, which also receives information from an anatomy estimation module 780 based on C-arm parameters 770 of the radiographic imaging system. The anatomy estimate 780 includes information about which anatomical features may be visible to the intraluminal ultrasound imaging device, and which anatomical features are likely not visible. This can significantly narrow the search parameters and reduce the computational burden on the ultrasound feature detection module 790. The ultrasound feature detection module 790 then identifies which anatomical features are actually seen within the ultrasound image 760, and their locations within the image frame. In some aspects, instead or in addition, the ultrasound feature detection module 790 may determine the motion of the imaged anatomical features within the image frame 760 when the roll orientation of the intraluminal imaging device is altered.

Based on this information, the ultrasound feature detection module 790 can disambiguate the roll angle (e.g., the angular orientation around the longitudinal axis) of the intraluminal ultrasound imaging device 110, reducing the two possible solutions g1 and g2 to a single disambiguated g value 755. This disambiguated 6-DoF pose 750 can then be used by a fusion/co-registration algorithm to accurately rotate, translate, and resize the ultrasound image 760 in order to overlay it onto the radiographic image 710, thus providing a fused or co-registered image that includes detailed views of both the interventional devices and the soft tissues around them.

It is noted that block diagrams are provided herein for exemplary purposes; a person of ordinary skill in the art will recognize myriad variations that nonetheless fall within the scope of the present disclosure. For example, block diagrams may show a particular arrangement of components, modules, services, steps, processes, or layers, resulting in a particular data flow. It is understood that some embodiments of the systems disclosed herein may include additional components, that some components shown may be absent from some embodiments, and that the arrangement of components may be different than shown, resulting in different data flows while still performing the methods described herein.

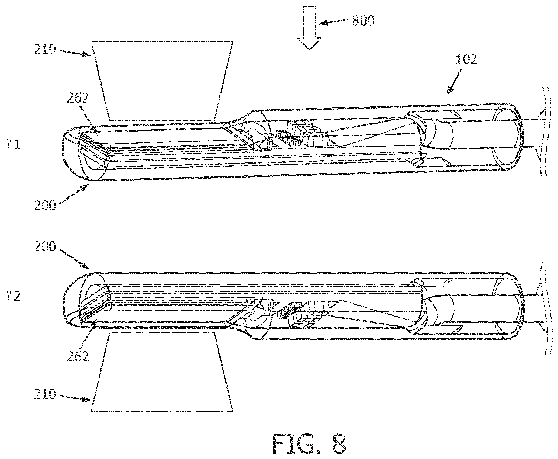

**Fig. 8** is a perspective view of the imaging assembly 102 of the ultrasound imaging device 110 in two substantially opposing orientations, according to aspects of the present disclosure. The imaging assembly 102 may include an imaging core 262 that is positioned within a tip member 200. In some embodiments, the tip member 200 is substantially cylindrical in shape except for the portion that houses the imaging core 262, which may be flat. In some embodiments, the imaging core 262 has a directional field of view 210. Fig. 8 shows the imaging assembly 102 in a "g1" orientation where the imaging core 262 faces upward with an upward field of view 210 and another imaging assembly 102 in a "g2" orientation where the imaging core 262 faces downward with a downward field of view 210. When being imaged along a direction 800 by a radiographic imaging device such as the radiographic imaging device 140, because the tip member is cylindrical in shape and of a small diameter, the radiographic imaging data do not contain enough information for the control and processing system 130 to determine the rotational orientation of the imaging assembly 102 precisely enough to adequate co-register the images. Sometimes the small dimensions of the imaging assembly 102 can make it more difficult for the control and processing system 130 to distinguish the "g1" and "g2" orientations. This makes it unreliable for the control and processing system 130 to determine the orientation of the imaging assembly 102 by the radiographic imaging data alone.

Advantageously, the systems and methods provided in the present disclosure eliminates the current unreliable orientation determination through radiographic imaging data, and instead introduces robust, repeatable steps for orientation determination, thereby facilitating accurate and fast fusion/co-registration of the ultrasound imaging data and the radiographic imaging data.

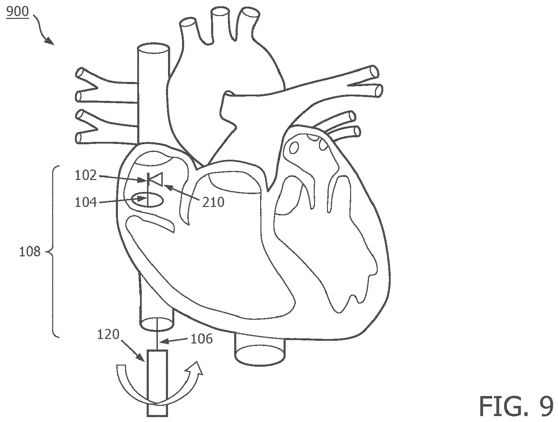

**Fig. 9** is a schematic, diagrammatic illustration of the ultrasound imaging device 110 with imaging assembly 102 positioned on distal portion 104 inserted into a heart 900 of a patient in a first orientation g1, and proximal portion 106 coupled to handle 120, according to embodiments of the present disclosure. When the imaging assembly 102 is in the first orientation g1, the field of view 210 of the imaging assembly 102 is pointed to the right of Fig. 9. Thus, the imaging assembly 102 is in a 6-DoF pose with a position (X, Y, Z) and yaw and pitch orientation (a, b) determinable from the radiographic imaging, and roll orientation of g1 that cannot be directly, reliably determined from the radiographic imaging.

The present disclosure provides systems and methods for determining the roll orientation based on a combination of the radiographic imaging and the ultrasound imaging.

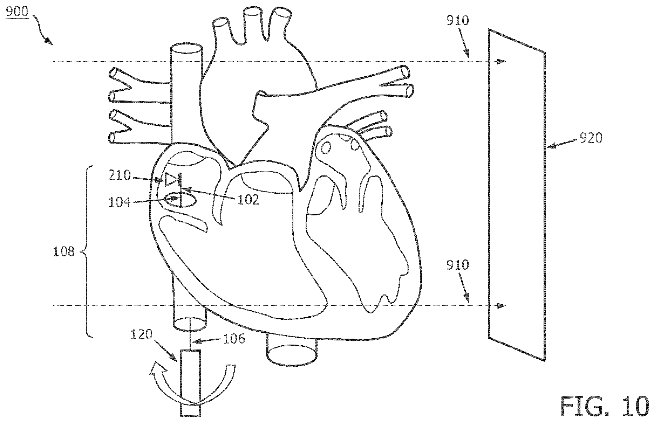

**Fig. 10** is a schematic, diagrammatic illustration of the imaging assembly 102 being inserted into the heart 900 in a second orientation different from the first orientation, according to aspects of the present disclosure. When the imaging assembly 102 is in the second orientation g2, the field of view 210 of the imaging assembly 102 is pointed to the left of Fig. 5. Thus, the imaging assembly 102 is in a 6-DoF pose with a position (X, Y, Z) and yaw and pitch orientation (a, b) determinable from the radiographic imaging, and roll orientation of g1 that cannot be directly, reliably determined from the radiographic imaging.

Because the flexible elongate member 108 is torsionally stiff, the flexible elongate member 108 and the imaging assembly 102 rotate with the handle 120. That is, by rotating the handle 120, the imaging assembly 102 can be rotated between the first orientation g1 shown in Fig. 9 and the second orientation g2 shown in Fig. 10. These two orientations may be difficult to distinguish in an X-ray image. Because the imaging assembly 102 of the ICE catheter is relatively small, the structures of the imaging assembly 102 in the ICE catheter appear the same/similar regardless of whether the imaging assembly is face-up or face-down in the X-ray image (e.g., whether it is in roll orientation g1 or g2). It is noted that intermediate orientations, in between completely face-up and completely face-down, are difficult to distinguish for the same reasons. For example, if the imaging assembly is oriented at 45 degrees to the direction of the X-rays 910 or the plane of the radiographic image 920, then it may be unclear from the X-ray image whether the orientation is 45 degrees up or 45 degrees down.

Figs. 9 and 10 show that the imaging assembly 102 enters into the heart via the inferior vena cava, suggesting that the imaging assembly 102 is inserted into the patient's body through the femoral vein. In some other examples, the imaging assembly 102 can enter into the heart via the superior vena cava and the imaging assembly 102 is inserted into the patient's body through the jugular vein, or through another appropriate vessel such as, but not limited to, the major arterial vessels with access to the cardiac chambers.

**Fig. 11A** is a schematic, diagrammatic overview, in block diagram form, of a training mode 1100 for the object detector 720 (see Fig. 7), according to aspects of the present disclosure. In the example shown in Fig. 11A, a set of training data 1105a that includes radiological images with labeled objects or features (e.g., intraluminal ultrasound imaging devices in known 6-DoF poses) is fed into an untrained object detector 1110a in an iterative training process that will be familiar to a person of ordinary skill in the art.

In particular, for object detection using convolutional neural networks, large numbers of sample images are manually annotated by experts to delineate the localizations of features of interest. The parameters of a network model (e.g., the weights at each artificial neuron) are initialized with initial values "A" that may be random values or with results from training on prior datasets. In an iterative process, the network is used to make detection inferences on the training images, the results are compared with the ground truth annotations, and an optimizer is used to adjust the network parameters "B" until a metric of detection accuracy is maximized.

Thus, an output of this training process 1100 is a trained object detector1110b, wherein the parameters "B" (e.g., weights) are optimized for detection of the features in the training videos 1105a.

Examples of object, detection, feature detection, object classification, or feature classification using machine learning models such as a neural network or convolutional neural network may for example be found in U.S. Provisional Application No. 63/437,206, filed January 5, 2023 (Atty. Dkt. No. 2022PF00672/44755.2335PV01), titled "MULTI-FRAME ULTRASOUND VIDEO WITH VIDEO-LEVEL FEATURE CLASSIFICATION BASED ON FRAME-LEVEL DETECTION", which is incorporated by reference as though fully set forth herein.

**Fig. 11B** is a schematic, diagrammatic overview, in block diagram form, of a validation mode 1102 for the object detector 720 (see Fig. 7), according to aspects of the present disclosure. In the validation mode, a set of hand-annotated validation videos (e.g., videos that include labels, bounding boxes, outlines, etc. around any intraluminal ultrasound imaging devices in known 6-DoF poses as identified by an expert, in each frame of each video) are fed into the trained object detector 1110b in order to determine whether human-identified features in the validation videos 1105b are detected by the trained object detector 1110b to a desired level of accuracy.

In some cases, performance of the trained object detector 1110b may be deemed to be below the desired level of accuracy. In this case, the parameters "B" (e.g., weights) of the trained object detector 1110b may be adjusted until detection accuracy on the validation dataset (or the validation dataset plus the training dataset) reaches the desired accuracy. In such cases, an output of the validation process may be the trained object detector 720, which may be identical to the trained object detector 1110b except for the adjusted parameters "C" (e.g., weights). In other cases, performance of the trained object detector 1110b may be deemed to be adequate, and so no adjustments to the parameters are made, and the trained object detector 720 may be identical to (e.g., uses the same weights as) the trained object detector 1110b.

The object detector 720 may implement or include any suitable type of learning network. For example, in some aspects, the object detector 720 could include a neural network, such as a convolutional neural network (CNN). In addition, the convolutional neural network may additionally or alternatively be or an encoder-decoder type network, or may utilize a backbone architecture based on other types of neural networks, such as an object detection network, classification network, etc. One nonlimiting example backbone network is the Darknet YOLO backbone, (e.g., Yolov3) which can be used for object detection. The CNN may for example include a set of N convolutional layers, where N may be any positive integer. Fully connected layers can be omitted when the CNN is a backbone. The CNN may also include max pooling layers and/or activation layers. Each convolutional layer may include a set of filters configured to extract features from an input (e.g., from the image frames 1105a or 1105b). The value N and the size of the filters may vary depending on the aspects. In some instances, the convolutional layers may utilize any non-linear activation function, such as for example a leaky rectified non-linear (ReLU) activation function and/or batch normalization. The max pooling layers gradually shrink the high-dimensional output to a dimension of the desired result (e.g., a bounding box of a detected feature).

Fully connected layers may be referred to as perception or perceptive layers. In some aspects, perception/perceptive and/or fully connected layers may be found in the object detector 720 (e.g., a multi-layer perceptron), to allow downstream processing (e.g., detection, segmentation, etc.). The object detector 720 may also include max pooling layers and/or activation layers. The max pooling layers gradually shrink the high-dimensional output to a dimension of the desired result (e.g., a positive identification of the probe head, along with a 5-DoF or 6-DoF pose).

These descriptions are included for exemplary purposes; a person of ordinary skill in the art will appreciate that other types of AI learning models, with features similar to or dissimilar to those described above, may be used instead or in addition, without departing from the spirit of the present disclosure.

**Fig. 11C** is a schematic, diagrammatic overview, in block diagram form, of an inference mode or clinical usage mode 1104 for the object detector 720, according to aspects of the present disclosure. In clinical usage, an X-ray video 1120 is fed to the trained and validated object detector 720 for analysis. In some cases, the X-ray video may be acquired and analyzed in real time or near-real time. In other cases, the X-ray video may be retrieved from memory, storage, or a network. The trained and validated object detector 720 then produces, as an output, an annotated version 1130 of the patient video 1120, which includes frame-level information about the 6-DoF pose of the intraluminal ultrasound imaging device.

Thus, in each real-time frame of the patient X-ray video, the object detector 720 is run to determine a localization and confidence value or values for the ultrasound probe. A confidence value can be determined as a normalized value in the range [0, 1], where 0 indicates lowest confidence, and 1 indicates highest confidence that the feature is present at that location.

Due to the uncertainty in the roll orientation g, in some aspects, the object detector 720 only a 5-DoF pose. In other aspects, the object detector 720 outputs a 6-DoF pose that includes two possible values g1 and g2 for the roll orientation g. As noted in Fig. 7, for some aspects of the present disclosure, identification and pose estimation of the intraluminal ultrasound imaging device does not require an ML model, but instead relies on classical image recognition and pattern matching techniques, including but not limited to thresholding, filtering, texture analysis, or combinations thereof, or other techniques known in the art.

**Fig. 12** is a diagram 1200 showing different views of the heart available as a function of C-arm geometry and pose, according to aspects of the present disclosure. It is noted that the terms "C-arm geometry" and "C-arm pose" are terms of art that collectively describe the full range of physical configurations and associated information of the C-arm. The diagram 1200 includes a cranial-caudal (CRA-CAU) axis 1210 of the patient and a right-left anterior oblique (RAO-LAO) axis 1220 of the patient, each ranging from -90° to +90°.

In an example, a 2-chamber view 1230 of the heart occurs when the C-arm of the fluoroscopic imaging system is set within the RAO/CAU quadrant of the diagram 1200. This 2-chamber view 1230 differentiates between the anterior and posterior leaflets of the tricuspid valve (TV), while providing maximal separation of the anterior and posterior TV papillary muscles, and providing an "en face" (e.g., fully perpendicular) view of the interatrial septum. In-plane features in this view include the coronary sinus, TV annulus, inferior vena cava, and right atrial appendage.

In an example, a 3-chamber view 1240 of the heart occurs when the C-arm of the fluoroscopic imaging system is set within the RAO/CRA quadrant of the diagram 1200. This 3-chamber 1240 view differentiates between the posterior and septal leaflets of the TV, while providing an overlapping view of the anterior and posterior TV papillary muscles, and an en face view of the coronary sinus. In-plane features in this view include the pulmonary valve, the TV annulus, the superior vena cava , and the right ventricle outflow tract.

In an example, a 4-chamber view 1250 of the heart occurs when the C-arm of the fluoroscopic imaging system is set within the LAO (or minimal RAO)/CRA portion of the diagram 1200. This 4-chamber view 1250 differentiates between the anterior and septal leaflets of the TV, while providing full separation between the right and left atria, and between the right and left ventricles. This view also provides an en face view of the right atrial appendage. In-plane features of this view include the interatrial septum, the TV annulus, and the interventricular septum.

In an example, a 1-chamber view 1260 of the heart occurs when the C-arm of the fluoroscopic imaging system is set within the LAO/CAU quadrant of the diagram 1200. This 1-chamber view 1260 is along the short-axis view of the heart, and appreciates all three leaflets of the TV, while elongating the right coronary path and the coronary sinus path.

It is noted that, although the fluoroscopic imaging system may visualize blood if a radiopaque contrast dye is present, it may not clearly visualize soft tissues in any of the views shown in Fig. 12. However, the ultrasound system can clearly visualize soft tissues within a limited distance (e.g., the field of view) of the ultrasound transducer array, but may not provide clear context as to where these features are located or oriented relative to the body of the patient. By registering (e.g., translating and/or rotating) the ultrasound image to overlay on the corresponding portion of the fluoroscopy image, the medical imaging system can provide improved visualization of the heart anatomy to a clinician (e.g., during an intracardiac intervention procedure). Thus, for proper fusion/registration, the 6-DoF pose of the intraluminal imaging device may be determined relative to the position and orientation of the radiographic imaging sensor, and/or the position and orientation of the fluoroscopy image and the ultrasound image must both be known relative to a coordinate system of the patient. More generally, if the 6-DoF pose of the fluoroscopy image is known in any coordinate system relative to the patient, to the radiographic imaging sensor, to the room, to the Earth, etc., then translating/rotating/scaling the ultrasound image into that same coordinate system will allow fusion/registration.

The C-arm geometry can thus provide details about what structures of the heart the ultrasound probe may and may not be able to see with the 5-DoF pose (X, Y, Z, a, b), in each of the candidate roll orientations g1 and g2. For example, if the C-arm is in the RAO/CAU quadrant of the diagram 1200 and the ultrasound probe is located within the right atrium and is in a ventral orientation (e.g., upward with respect to a patient lying on a table), then depending on the procedure, the ultrasound probe should be able to see one or more of the aortic valve, the mitral valve, or the tricuspid valve, whereas if the ultrasound probe is pointed dorsally (e.g., downward with respect to the patient), then the probe should be able to see the anatomy beyond the wall of the inferior-superior vena cava. Thus, the C-arm orientation can provide information that significantly narrows the search parameters for object/anatomy feature identification within the ultrasound video stream. The c-arm geometry and pose can tell the system that the intended anatomy of interest is, for example, the left atrial appendage (LAA). Thus, in all the possible permutations and combinations of possible views that the ultrasound can see, the search space of the anatomy to be identified can be limited to the LAA or nearby structures. Once detected, this confirms that the probe is indeed visualizing this anatomy and can thus disambiguate the roll. The ultrasound-based detection/identification can do this independently as well, but an additional source of information can make the disambiguation more robust.

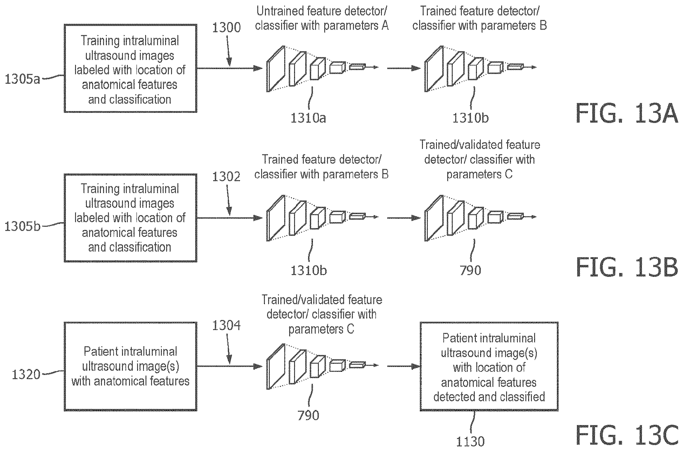

**Fig. 13A** is a schematic, diagrammatic overview, in block diagram form, of a training mode 1300 for the ultrasound feature detector 790 (see Fig. 7), according to aspects of the present disclosure. In the example shown in Fig. 13A, a set of training data 1305a that includes ultrasound images with labeled features (e.g., particular structures of the heart, as seen from inside the heart by an ICE probe) is fed into an untrained object detector 1310a in an iterative training process that will be familiar to a person of ordinary skill in the art.

In particular, for object detection using convolutional neural networks, large numbers of sample images are manually annotated by experts to delineate the localizations of features of interest. The parameters of a network model (e.g., the weights at each artificial neuron) are initialized with initial values "A" that may be random values or with results from training on prior datasets. In an iterative process, the network is used to make detection inferences on the training images, the results are compared with the ground truth annotations, and an optimizer is used to adjust the network parameters "B" until a metric of detection accuracy is maximized.

Thus, an output of this training process 1300 is a trained feature detector1310b, wherein the parameters "B" (e.g., weights) are optimized for detection of the features in the training videos 1305a. **Fig. 13B** is a schematic, diagrammatic overview, in block diagram form, of a validation mode 1302 for the ultrasound feature detector 790 (see Fig. 7), according to aspects of the present disclosure. In the validation mode, a set of hand-annotated validation videos (e.g., videos that include labels, bounding boxes, outlines, etc. around known features of the heart, as identified by an expert, in each frame of each video) are fed into the trained object detector 1310b in order to determine whether human-identified features in the validation videos 1305b are detected by the trained feature detector 1310b to a desired level of accuracy.

In some cases, performance of the trained feature detector 1310b may be deemed to be below the desired level of accuracy. In this case, the parameters "B" (e.g., weights) of the trained feature detector 1310b may be adjusted until detection accuracy on the validation dataset (or the validation dataset plus the training dataset) reaches the desired accuracy. In such cases, an output of the validation process may be the trained feature detector 790, which may be identical to the trained feature detector 1310b except for the adjusted parameters "C" (e.g., weights). In other cases, performance of the trained feature detector 1310b may be deemed to be adequate, and so no adjustments to the parameters are made, and the validated feature detector 790 may be identical to (e.g., uses the same weights as) the trained feature detector 1310b.

The feature detector 790 may implement or include any suitable type of classical image recognition techniques, or may include a learning network. For example, in some aspects, the feature detector 790 could include a neural network, such as a convolutional neural network (CNN).

**Fig. 13C** is a schematic, diagrammatic overview, in block diagram form, of an inference mode or clinical usage mode 1304 for the ultrasound feature detector 790, according to aspects of the present disclosure. In clinical usage, an ultrasound video 1320 (e.g., the real-time image stream from an ICE probe) is fed to the trained and validated feature detector 790 for analysis. In some cases, the ultrasound video may be acquired and analyzed in real time or near-real time. In other cases, the ultrasound video may be retrieved from memory, storage, or a network. The trained and validated feature detector 790 then produces, as an output, an annotated version 1330 of the patient video 1320, which includes frame-level information about the detected anatomical features.

Thus, in each real-time frame of the patient ultrasound video, the ultrasound feature detector 790 is run to determine localizations, classifications, and confidence values for the anatomical features visible within the ultrasound probe's field of view. A confidence value can for example be determined as a normalized value in the range [0, 1], where 0 indicates lowest confidence, and 1 indicates highest confidence that the feature is present at that location.

The identification of specific anatomical features visible to the ultrasound probe, coupled with the 5-DoF pose of the ultrasound probe (X, Y, Z, a, b) in a coordinate system of the X-ray image stream, can be used to disambiguate the two candidate roll orientations g1 and g2 into a single known value g, as described above. The resulting 6-DoF pose then allows a fusion/co-registration algorithm to place (e.g., translate, rotate, and resize) the ultrasound image within the X-ray image.

It is noted that for some aspects of the present disclosure, feature identification within the intraluminal ultrasound images may not require an ML model, may but instead rely on classical image recognition and pattern matching techniques, including but not limited to thresholding, filtering, texture analysis, or combinations thereof, or other techniques known in the art.

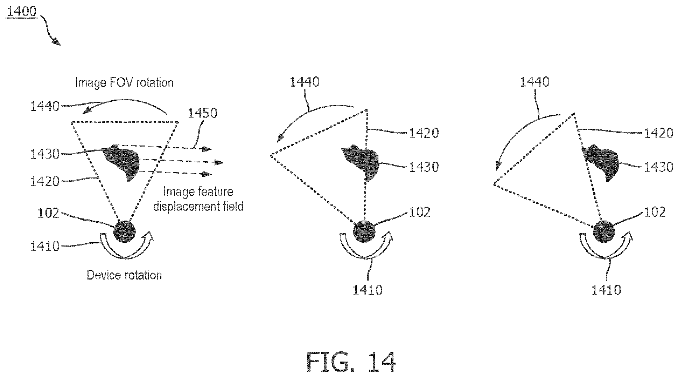

**Fig. 14** is a schematic, diagrammatic view 1400 of the displacement 1450 of an image feature 1420 in the field of view 1420 of an intraluminal ultrasound imaging assembly 102 during roll rotational motion 1410, according to aspects of the present disclosure. The field of view 1420 is shaped as a cone in the example in Fig. 14, but any suitable field of view of the intraluminal ultrasound imaging assembly 102 is contemplated. When the imaging assembly 102 undergoes rotational motion 1410 in a particular direction (e.g., counterclockwise when looking end-on at the proximal end of the intraluminal imaging device), the field 1420 of the imaging assembly 102 undergoes rotational motion 1440 in the same direction, such that an ultrasound image feature 1420 (whether identified or not) appears to move in the opposite direction, until the image feature 1430 leaves the field of view 1420. Such motion effects are consistent - e.g., counterclockwise motion 1410 of the imaging assembly 102 will result in clockwise apparent motion 1450 of the image feature 1430, whereas clockwise motion 1410 of the imaging assembly 102 will result in counterclockwise apparent motion 1450 of the image feature 1430. Furthermore, clockwise rotation of the handle 120 of the ultrasound imaging device 112 will result in clockwise rotation of the imaging assembly 102, and counterclockwise rotation of the handle 120 will result in counterclockwise rotation of the imaging assembly.

The direction of the apparent rotational motion 1430 of the image feature can be used trigonometrically, to disambiguate the roll orientation g from the candidate roll orientations g1 and g2, because an example co-registered image stream would show the image feature 1430 moving in a particular direction (e.g., left) if the imaging assembly 102 were in a face-down orientation, and the same example co-registered image stream would show the image feature moving in a 180-degree opposite direction. Thus, in some aspects, the disambiguation of g from g1 and g2 can take place using only such motion analysis, without identification of the features (e.g., features of heart anatomy) visible in the ultrasound image. In other aspects, the disambiguation may involve only feature identification from the ultrasound image, and in still other embodiments, the disambiguation may involve a combination of motion analysis and feature identification. In another aspect, this apparent motion of the features can also quantify the exact amount of roll (e.g., in degrees) as the probe handle is rotated about the longitudinal axis.

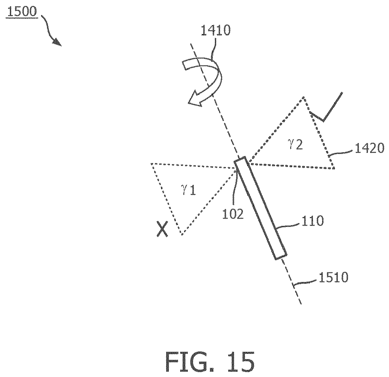

**Fig. 15** is a schematic, diagrammatic view of a roll angle disambiguation 1500, according to aspects of the present disclosure. In the example shown in Fig. 15, the rotational motion 1410 of the ultrasound imaging assembly 102 around the longitudinal axis 1510 of the intraluminal imaging device 110 has permitted the pose determination system to disambiguate the roll orientation of the imaging assembly 102 as described above, such that one candidate roll orientation g1 has been rejected or disproven, whereas the other candidate roll orientation g2 has been accepted or proven, such that g = g2. One of the first rotational orientation glor the second rotational orientation g2 (in this example, the second rotational orientation g2) around the longitudinal axis is selected/identified for the intraluminal imaging assembly. For example, the selected/identified second rotational orientation g2 can be referred to as the actual or true rotational orientation, to distinguish it from the candidate/possible orientations. An x is provided next to the field of view of rotational orientation g1 because it is not selected. A checkmark is provided next to the field of view of rotational orientation g2 because it is selected. The pose determination system can now pass a complete 6-DoF pose (X, Y, Z, a, b, selected/identified g) to fusion/co-registration step 670 (see Fig. 6), thus allowing the ultrasound image stream to be accurately overlaid onto the X-ray image stream.

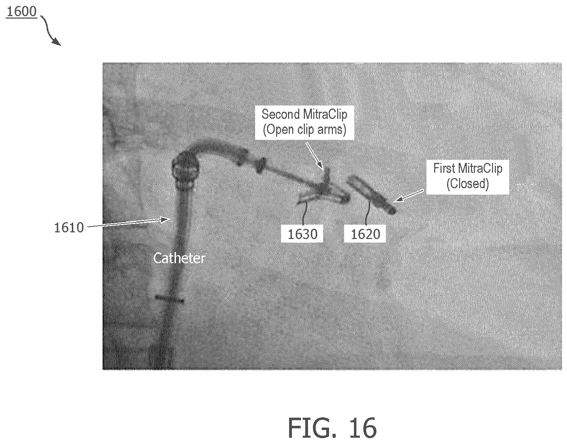

**Fig. 16** is an X-ray image 1600 of an ongoing intracardiac interventional procedure, taken from outside the body, according to aspects of the present disclosure. Visible are an intervention catheter 1610, a first mitral valve clip 1620 that has been placed in a closed position on a leaflet of the mitral valve, and a second mitral valve clip 1630, still coupled to the intervention catheter 1610, that has not yet been placed and remains in the open position. Notably, soft tissue structures such as the mitral valve leaflets, are not distinguishable in the X-ray image 1600, even though visualizing the leaflets may be critical for engaging them correctly with the mitral valve clips 1620 and 1630. Conversely, ultrasound images may clearly visualize soft tissue structures such as the mitral valve leaflets, but may poorly visualize solid structures such as the mitral valve clips, and may provide little positional or orientational context as to the location being imaged. Thus, there is a need for co-registered X-ray and intracardiac ultrasound image streams that provide such context, while also clearly imaging both interventional devices and the soft tissues with which they interact.

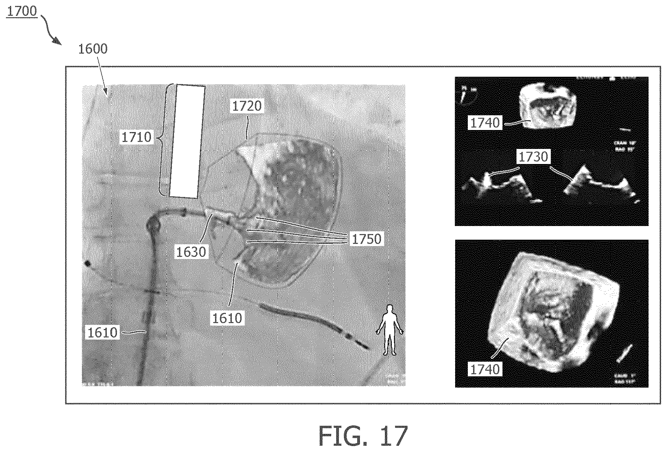

**Fig. 17** is a screen display 1700 including a fused, co-registered X-ray and intraluminal image of an ongoing intracardiac interventional procedure, according to aspects of the present disclosure. In an example, the X-ray and ultrasound images are co-registered and displayed according to steps 670-685 of Fig. 6, although other steps may be used instead or in addition. Visible are the X-ray image 1600, interventional catheter 1610, first (closed) mitral clip 1620, second (open) mitral clip 1630, and an intraluminal ultrasound imaging device 1710 with a field of view 1720. The intraluminal ultrasound imaging device 1710 can be representative of an ICE catheter. Other examples of an intraluminal ultrasound imaging device 1710 include a TEE probe or mini-TEE probe. Also visible are two 2D ultrasound cross-sectional images 1730 (e.g., can be representative of ultrasound data obtained by intraluminal ultrasound imaging device 1710, such as the ICE catheter), and two 3D rotations 1740 of a 3D ultrasound image (e.g., can be representative of ultrasound data obtained by intraluminal ultrasound imaging device 1710, such as the ICE catheter). Also visible is the registered ultrasound image 1750 (e.g., can be representative of ultrasound data obtained by intraluminal ultrasound imaging device 1710, such as the ICE catheter) overlaid at the correct position, rotation, and scale on the X-ray image 1600. The registered ultrasound image 1750 in the example of Fig. 17 is 3D ultrasound image/image data. The registered ultrasound image 1750 can be a 2D ultrasound image/image data in some instances. Visible in the registered ultrasound image are the flaps 1750 of the mitral valve.

As can be seen in Fig. 17, the fused, co-registered image screen display 1700 provides spatial context, visibility of imaging probes and interventional devices, and visibility of soft tissue features for which the positioning of interventional devices (e.g., the mitral valve clips 1620, 1630) may be critical.

Examples of image fusion and/or co-registration techniques may for example be found in U.S. Patent No. 10,238,361, titled "Combination of ultrasound and x-ray systems", U.S. Patent No. 10,157,491, titled "Real-time scene-modeling combining 3D ultrasound and 2D X-ray imagery", which are incorporated by reference as though fully set forth herein.

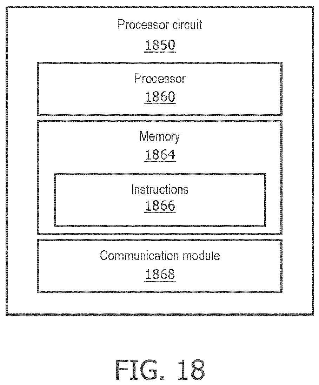

**Fig. 18** is a schematic diagram of a processor circuit 1850, according to embodiments of the present disclosure. The processor circuit 1850 may be implemented in the medical imaging system 100, the pose estimation system 700, or other devices or workstations (e.g., third-party workstations, network routers, etc.), or on a cloud processor or other remote processing unit, as necessary to implement the method. As shown, the processor circuit 1850 may include a processor 1860, a memory 1864, and a communication module 1868. These elements may be in direct or indirect communication with each other, for example via one or more buses.

The processor 1860 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 1860 may also comprise another hardware device, a firmware device, or any combination thereof configured to perform the operations described herein. The processor 1860 may also be implemented as a combination of computing devices, e.g., a combination of a DSP and a microprocessor, a plurality of microprocessors, one or more microprocessors in conjunction with a DSP core, or any other such configuration.

The memory 1864 may include a cache memory (e.g., a cache memory of the processor 1860), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 1864 includes a non-transitory computer-readable medium. The memory 1864 may store instructions 1866. The instructions 1866 may include instructions that, when executed by the processor 1860, cause the processor 1860 to perform the operations described herein. Instructions 1866 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 1868 can include any electronic circuitry and/or logic circuitry to facilitate direct or indirect communication of data between the processor circuit 1850, and other processors or devices. In that regard, the communication module 1868 can be an input/output (I/O) device. In some instances, the communication module 1868 facilitates direct or indirect communication between various elements of the processor circuit 1850 and/or the medical imaging system 100. The communication module 1868 may communicate within the processor circuit 1850 through numerous methods or protocols. Serial communication protocols may include but are not limited to United States Serial Protocol Interface (US SPI), Inter-Integrated Circuit (I²C), Recommended Standard 232 (RS-232), RS-485, Controller Area Network (CAN), Ethernet, Aeronautical Radio, Incorporated 429 (ARINC 429), MODBUS, Military Standard 1553 (MIL-STD-1553), or any other suitable method or protocol. Parallel protocols include but are not limited to Industry Standard Architecture (ISA), Advanced Technology Attachment (ATA), Small Computer System Interface (SCSI), Peripheral Component Interconnect (PCI), Institute of Electrical and Electronics Engineers 488 (IEEE-488), IEEE-1284, and other suitable protocols. Where appropriate, serial and parallel communications may be bridged by a Universal Asynchronous Receiver Transmitter (UART), Universal Synchronous Receiver Transmitter (USART), or other appropriate subsystem.

External communication (including but not limited to software updates, firmware updates, preset sharing between the processor and central server, or readings from the ultrasound or X-ray detectors) may be accomplished using any suitable wireless or wired communication technology, such as a cable interface such as a universal serial bus (USB), micro USB, Lightning, or FireWire interface, Bluetooth, Wi-Fi, ZigBee, Li-Fi, or cellular data connections such as 2G/GSM (global system for mobiles) , 3G/UMTS (universal mobile telecommunications system), 4G, long term evolution (LTE), WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

As will be readily appreciated by those having ordinary skill in the art after becoming familiar with the teachings herein, the pose determination system disclosed herein advantageously permits determination of the roll angle of a small, rotationally symmetrical intracardiac probe such as an ICE catheter, so that registration and/or image fusion algorithms can be employed for these types of probes, thus enabling faster, more precise placement of intraluminal (e.g., intracardiac) interventional devices, with higher confidence in the results, and better traceability.

This technology can be utilized for image fusion on diverse platforms, including but not limited to Philips EchoNavigator, Philips Azurion, Zenition, and EPIQ. The inventive elements described herein, and their combined technical effects, can be detected by visually observing a system which allows for the fusion of intracardiac (or other intraluminal) ultrasound data with X-ray fluoroscopy, or by observing the X-ray images of the ultrasound transducer to see if the image represents symmetric transducer head.

A number of variations are possible on the examples and embodiments described above. For example, the technology could be applied to imaging of organs or anatomical systems other than the heart. The technology is applicable to other extraluminal (outside-the-body) imaging systems, including but not limited to computer tomography (CT), positron emission tomography (PET), magnetic resonance imaging (MRI), functional MRI (fMRI), and others. Similarly, the technology can be applicable to other intraluminal imaging systems, including not only TEE and ICE probes, but also intravascular ultrasound (IVUS), optical coherence tomography (OCT), and others. Other specific steps or combinations of methods for estimating the 5-DoF and 6-DoF pose may be employed instead or in addition to those described herein, without departing from the spirit of the present disclosure.

Accordingly, the logical operations making up the embodiments of the technology described herein are referred to variously as operations, steps, blocks, objects, elements, components, or modules. It should be understood that these may be performed in any order, unless explicitly claimed otherwise or a specific order is inherently necessitated by the claim language.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the pose estimation system. Connection references, e.g., attached, coupled, connected, joined, or "in communication with" are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary embodiments of the pose estimation system as defined in the claims. Although various embodiments of the claimed subject matter have been described above with a certain degree of particularity, or with reference to one or more individual embodiments, those skilled in the art could make numerous alterations to the disclosed embodiments without departing from the spirit or scope of the claimed subject matter.

Still other embodiments are contemplated. It is intended that all matter contained in the above description and shown in the accompanying drawings shall be interpreted as illustrative only of particular embodiments and not limiting. Changes in detail or structure may be made without departing from the basic elements of the subject matter as defined in the following claims.

## Claims

1. A system, comprising:
a processor circuit configured to:
receive extraluminal imaging data obtained by an extraluminal imaging device, wherein the extraluminal imaging data depicts:
an intraluminal imaging device positioned within a patient body; and
an intraluminal imaging assembly of the intraluminal imaging device in either a first rotational orientation or a second rotational orientation around a longitudinal axis of the intraluminal imaging device;
receive intraluminal imaging data obtained by the intraluminal imaging device;
receive an extraluminal imaging device parameter associated the extraluminal imaging device obtaining the extraluminal imaging data;
identify, based on the intraluminal imaging data and the extraluminal imaging device parameter, an anatomical feature depicted in the intraluminal imaging data;
select, based on the anatomical feature, one of the first rotational orientation or the second rotational orientation for the intraluminal imaging assembly;
perform image fusion of the intraluminal imaging data and the extraluminal imaging data based on the selected rotational orientation of the imaging assembly; and
output, to a display in communication with the processor circuit, the co-registered intraluminal imaging data and extraluminal imaging data.

2. The system of claim 1, wherein, to select one of the first rotational orientation or the second rotational orientation, the processor circuit is configured to:
identify an anatomical feature depicted in the intraluminal imaging data.

3. The system of claim 1, the processor circuit is configured to select one of the first rotational orientation or the second rotational orientation based on motion of the anatomical feature in the intraluminal imaging data during rotation of the intraluminal imaging assembly around the longitudinal axis.

4. The system of claim 1, wherein the processor circuit is configured to use a first machine learning model to select one of the first rotational orientation or the second rotational orientation.

5. The system of claim 1,
wherein the intraluminal imaging device comprises intra-cardiac echocardiography (ICE) catheter, and
wherein the intraluminal imaging assembly comprises an ultrasound transducer array.

6. The system of claim 1, wherein the wherein the extraluminal imaging device comprises a radiographic imaging device.

7. The system of claim 6, wherein the radiographic imaging device comprises an x-ray imaging device.

8. The system of claim 1, wherein the extraluminal imaging device parameter comprises at least one of:
a pose of the extraluminal imaging device; or
a geometry of the extraluminal imaging device.

9. The system of claim 8, wherein the extraluminal imaging device parameter comprises an angle of the extraluminal imaging device along a cranial-caudal axis or a right-left anterior oblique axis.

10. The system of claim 1, wherein the processor circuit is configured to:
determine, based on the radiographic imaging data:
a position of the intraluminal imaging assembly in the radiographic imaging data;
a rotational orientation of the intraluminal imaging assembly around a transverse axis of the intraluminal imaging device; and
a rotational orientation of the intraluminal imaging assembly around a vertical axis of the intraluminal imaging device; and
co-register the intraluminal imaging data and the extraluminal imaging data based on the position of the intraluminal imaging assembly, the rotational orientation of the intraluminal imaging assembly around the transverse axis, and the rotational orientation of the intraluminal imaging assembly around the vertical axis.

11. The system of claim 10, wherein the processor circuit is configured to use a second machine learning model to determine the position of the intraluminal imaging assembly, the rotational orientation of the intraluminal imaging assembly around the transverse axis, and the rotational orientation of the intraluminal imaging assembly around the vertical axis.

12. The system of claim 1,
wherein, to co-register the intraluminal imaging data and the extraluminal imaging data, the processor circuit is configured to overlay the intraluminal imaging data on the extraluminal imaging data,
wherein, to output the co-registered intraluminal imaging data and extraluminal imaging data, the processor circuit is configured to output the intraluminal imaging data overlaid the extraluminal imaging data.

13. The system of claim 1, further comprising the intraluminal imaging device.

14. The system of claim 13,
wherein the processor circuit is configured for communication with the intraluminal imaging device, and
wherein the processor circuit is configured to control the intraluminal imaging assembly to obtain the intraluminal imaging data.

15. The system of claim 1, further comprising the extraluminal imaging device.

16. The system of claim 15,
wherein the processor circuit is configured for communication with the extraluminal imaging device, and
wherein the processor circuit is configured to control the extraluminal imaging device to obtain the extraluminal imaging data.

17. A system, comprising:
an intracardiac echocardiography (ICE) catheter configured to be positioned within a heart of a patient, wherein the ICE catheter comprises an ultrasound transducer array configured to obtain ultrasound imaging data of the heart while the ICE catheter is positioned within the heart;
a radiographic imaging device configured to obtain radiographic imaging data of the heart, wherein the radiographic imaging device comprises at least one of a pose or a geometry associated with obtaining the radiographic imaging data; and
a processor circuit configured for communication with the ultrasound transducer array and the radiographic imaging device, wherein the processor circuit is configured to:
receive the radiographic imaging data from the radiographic imaging device, wherein the radiographic imaging data depicts the ICE catheter while the ICE catheter is positioned within the heart;
determine, based on the radiographic imaging data, a first candidate rotational orientation and a second candidate rotational orientation of the ultrasound transducer array around a longitudinal axis of the ICE catheter as a result of a size of the ICE catheter preventing determination of an actual rotational orientation of the ultrasound transducer array around the longitudinal axis based only the radiographic imaging data;
receive the ultrasound imaging data from the ultrasound transducer array;
receive at least one of the pose or the geometry of the radiographic imaging device;
identify, based on the ultrasound imaging data and at least one of the pose or the geometry, an anatomical feature depicted in the ultrasound imaging data;
select, based on the anatomical feature or motion of the anatomical feature, one of the first candidate rotational orientation or the second candidate rotational orientation as the actual rotational orientation of the ultrasound transducer array around the longitudinal axis;
perform image fusion of the ultrasound imaging data and the radiographic imaging data based on the actual rotational orientation of the ultrasound transducer array around the longitudinal axis; and
output, to a display in communication with the processor circuit, the co-registered ultrasound imaging data and radiographic imaging data.
